(19) Europäisches Patentamt
European Patent Office
Office européen des brevets

(11) **EP 3 954 696 A1**

(12) **EUROPEAN PATENT APPLICATION**
published in accordance with Art. 153(4) EPC

(43) Date of publication:
**16.02.2022 Bulletin 2022/07**

(21) Application number: **20787324.1**

(22) Date of filing: **09.04.2020**

(51) International Patent Classification (IPC):
***C07K 1/22*** *(2006.01)*     ***C07K 16/00*** *(2006.01)*

(52) Cooperative Patent Classification (CPC):
**C07K 1/22; C07K 16/00**

(86) International application number:
**PCT/JP2020/015904**

(87) International publication number:
**WO 2020/209318 (15.10.2020 Gazette 2020/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.04.2019 JP 2019074738**

(71) Applicant: **CHUGAI SEIYAKU KABUSHIKI KAISHA Tokyo 115-8543 (JP)**

(72) Inventors:
• **WAKABAYASHI, Tetsuya**
**Gotemba-shi, Shizuoka 412-8513 (JP)**
• **SHIMIZU, Yuichiro**
**Synapse, 138623 (SG)**
• **FUKUNAGA, Masahiro**
**Tokyo 115-8543 (JP)**
• **MAJIMA, Eiji**
**Higashikagawa-shi, Kagawa 769-2604 (JP)**

(74) Representative: **Vossius & Partner Patentanwälte Rechtsanwälte mbB Siebertstraße 3 81675 München (DE)**

(54) **METHOD FOR PURIFYING FC REGION-MODIFIED ANTIBODY**

(57) The present inventors discovered affinity purification resins having sufficient binding affinity for Fc region variants with reduced binding to Protein A. Specifically, immunoglobulins containing an Fc region variant having reduced binding to Protein A could be purified using a Protein A-modified ligand containing a structure in which the amino acids of the C-domain have been substituted as an Fc ligand.

EP 3 954 696 A1

SEQUENCE LISTING

<110>   CHUGAI SEIYAKU KABUSHIKI KAISHA
        ProteNova CO., LTD.

<120>   METHOD OF PURIFYING FC-MODIFIED ANTIBODY

<130>   C1-A1722P

<150>   JP 2019-074738
<151>   2019-04-10

<160>   62

<170>   PatentIn version 3.5

<210>   1
<211>   58
<212>   PRT
<213>   Artificial Sequence

<220>
<223>   modified C domain of staphylococcal protein A

<400>   1

Ala Asp Asn Lys Phe Asn Lys Glu Gln Gln Asn Ala Phe Tyr Glu Ile
1               5                   10                  15


Leu His Leu Pro Asn Leu Thr Glu Glu Gln Arg Asn Ala Phe Ile Gln
            20                  25                  30


Ser Leu Lys Asp Asp Pro Ser Val Ser Lys Glu Ile Leu Ala Glu Ala
            35                  40                  45


Lys Lys Leu Asn Asp Ala Gln Ala Pro Lys
        50                  55


<210>   2
<211>   58
<212>   PRT
<213>   Staphylococcus aureus

<400>   2

Val Asp Asn Lys Phe Asn Lys Glu Gln Gln Asn Ala Phe Tyr Glu Ile
1               5                   10                  15


Leu His Leu Pro Asn Leu Asn Glu Glu Gln Arg Asn Ala Phe Ile Gln
            20                  25                  30


Ser Leu Lys Asp Asp Pro Ser Gln Ser Ala Asn Leu Leu Ala Glu Ala
            35                  40                  45


Lys Lys Leu Asn Asp Ala Gln Ala Pro Lys
        50                  55

```
<210>  3
<211>  58
<212>  PRT
<213>  Artificial Sequence

<220>
<223>  modified immunoglobulin-binding domain

<400>  3


Ala Asp Asn Ala Phe Asn Thr Glu Gln Gln Asn Ala Phe Tyr Glu Ile
1               5                   10                  15


Leu His Leu Pro Asn Leu Thr Glu Glu Gln Arg Asn Ala Phe Ile Gln
            20                  25                  30


Ser Leu Arg Asp Asp Pro Ser Val Ser Lys Glu Ile Leu Ala Glu Ala
            35                  40                  45


Lys Lys Leu Asn Asp Ala Gln Ala Pro Lys
      50                  55



<210>  4
<211>  58
<212>  PRT
<213>  Artificial Sequence

<220>
<223>  modified immunoglobulin-binding domain

<400>  4


Ala Asp Asn Ala Phe Asn Thr Glu Gln Gln Asn Ala Phe Tyr Glu Ile
1               5                   10                  15


Leu His Leu Pro Asn Leu Thr Glu Glu Gln Arg Asn Ala Phe Ile Gln
            20                  25                  30


Ser Leu Arg Asp Asp Pro Ser Lys Ser Lys Lys Ile Leu Lys Glu Ala
            35                  40                  45


Lys Lys Leu Asn Lys Ala Gln Ala Pro Lys
      50                  55



<210>  5
<211>  58
<212>  PRT
<213>  Artificial Sequence

<220>
<223>  modified immunoglobulin-binding domain

<400>  5
```

```
Ala Asp Asn Ala Phe Asn Thr Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Arg Asp Asp Pro Ser Val Ser Ala Glu Ile Leu Ala Glu Ala
        35                  40                  45

Arg Arg Leu Asn Asp Ala Gln Ala Pro Arg
    50                  55
```

```
<210>  6
<211>  105
<212>  PRT
<213>  Artificial Sequence

<220>
<223>  IgG CH3 region SG1080

<400>  6
```

```
Gly Gln Arg Arg Glu Pro Gln Val Tyr Thr Leu Pro Pro Ser Arg Glu
1               5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Ala His Tyr
                85                  90                  95

Thr Arg Lys Glu Leu Ser Leu Ser Pro
            100                 105
```

```
<210>  7
<211>  105
<212>  PRT
<213>  Artificial Sequence

<220>
<223>  IgG CH3 region PK55
```

<400> 7

Gly Gln Arg Arg Glu Pro Gln Val Tyr Thr Leu Pro Pro Ser Arg Glu
1                   5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
        50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro
            100                 105

<210> 8
<211> 105
<212> PRT
<213> Artificial Sequence

<220>
<223> IgG CH3 region PK56

<400> 8

Gly Gln Arg Arg Glu Pro Gln Val Tyr Thr Leu Pro Pro Ser Arg Glu
1                   5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
        50                  55                  60

Phe Leu Tyr Ser Lys Leu Thr Val Arg Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro
                100                 105


<210>  9
<211>  105
<212>  PRT
<213>  Artificial Sequence

<220>
<223>  IgG CH3 region

<400>  9

Gly Gln Arg Arg Glu Pro Gln Val Tyr Thr Leu Pro Pro Ser Arg Glu
1                   5                   10                  15


Glu Met Thr Lys Asn Gln Val Ser Leu Thr Cys Leu Val Lys Gly Phe
                20                  25                  30


Tyr Pro Ser Asp Ile Ala Val Glu Trp Glu Ser Asn Gly Gln Pro Glu
                35                  40                  45


Asn Asn Tyr Lys Thr Thr Pro Pro Val Leu Asp Ser Asp Gly Ser Phe
            50                  55                  60


Phe Leu Tyr Ser Lys Leu Thr Val Asp Lys Ser Arg Trp Gln Gln Gly
65                  70                  75                  80


Asn Val Phe Ser Cys Ser Val Met His Glu Ala Leu His Ala His Thr
                85                  90                  95


Thr Arg Lys Glu Leu Ser Leu Ser Pro
                100                 105


<210>  10
<211>  105
<212>  PRT
<213>  Artificial Sequence

<220>
<223>  IgG CH3 region TT91

<400>  10

Gly Gln Arg Arg Glu Pro Gln Val Tyr Thr Leu Pro Pro Ser Arg Glu
1                   5                   10                  15


Glu Met Thr Lys Asn Gln Val Ser Leu Thr Cys Leu Val Lys Gly Phe
                20                  25                  30


Tyr Pro Ser Asp Ile Ala Val Glu Trp Glu Ser Asn Gly Gln Pro Glu
                35                  40                  45

```
Asn Asn Tyr Lys Thr Thr Pro Pro Val Leu Asp Ser Asp Gly Ser Phe
        50              55              60

Phe Leu Tyr Ser Lys Leu Thr Val Asp Lys Ser Arg Trp Gln Gln Gly
65              70              75                      80

Asn Val Phe Ser Cys Ser Val Leu His Glu Ala Leu His Ala His Thr
                85              90              95

Thr Arg Lys Glu Leu Ser Leu Ser Pro
            100             105
```

```
<210>  11
<211>  105
<212>  PRT
<213>  Artificial Sequence

<220>
<223>  IgG CH3 region

<400>  11
```

```
Gly Gln Arg Arg Glu Pro Gln Val Tyr Thr Leu Pro Pro Ser Arg Glu
1               5                   10                  15

Glu Met Thr Lys Asn Gln Val Ser Leu Thr Cys Leu Val Lys Gly Phe
            20              25                      30

Tyr Pro Ser Asp Ile Ala Val Glu Trp Glu Ser Asn Gly Gln Pro Glu
        35              40                      45

Asn Asn Tyr Lys Thr Thr Pro Pro Val Leu Asp Ser Asp Gly Ser Phe
        50              55              60

Phe Leu Tyr Ser Lys Leu Thr Val Asp Lys Ser Arg Trp Gln Gln Gly
65              70              75                      80

Asn Val Phe Ser Cys Ser Val Leu His Glu Ala Leu His Ala His Tyr
                85              90              95

Thr Arg Lys Glu Leu Ser Leu Ser Pro
            100             105
```

```
<210>  12
<211>  328
<212>  PRT
<213>  Artificial Sequence

<220>
<223>  IgG heavy chain constant region SG1074
```

<400> 12

```
Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
        100             105             110

Pro Ala Pro Glu Leu Leu Asn Gly Pro Ser Val Phe Leu Phe Pro Pro
        115             120             125

Lys Pro Lys Asp Thr Leu Met Ile Ser Arg Thr Pro Glu Val Thr Cys
    130             135             140

Val Val Val Asp Val Ser Asp Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Leu Tyr Asn Ser Thr Tyr Arg Val Val Ser Val Leu Thr Val Leu
            180             185             190

His Arg Asp Trp Leu Asn Gly Lys Glu Tyr Lys Cys Lys Val Ser Asn
        195             200             205

Thr Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240
```

```
Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
                260                 265                 270

Asn Tyr Lys Thr Thr Pro Pro Val Leu Asp Ser Asp Gly Ser Phe Phe
                275                 280                 285

Leu Tyr Ser Lys Leu Thr Val Asp Lys Ser Arg Trp Gln Gln Gly Asn
        290                 295                 300

Val Phe Ser Cys Ser Val Leu His Glu Ala Leu His Ala His Thr Thr
305                 310                 315                 320

Arg Lys Glu Leu Ser Leu Ser Pro
                325
```

```
<210>   13
<211>   328
<212>   PRT
<213>   Artificial Sequence

<220>
<223>   IgG heavy chain constant region SG1077

<400>   13
```

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
                20                  25                  30

Phe Pro Glu Pro Val Thr Val Ser Trp Asn Ser Gly Ala Leu Thr Ser
                35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
                100                 105                 110

Pro Ala Pro Glu Leu Trp Asn Gly Pro Ser Val Phe Leu Phe Pro Pro
```

                    115                    120                    125

        Lys Pro Lys Asp Thr Leu Met Ile Ser Arg Thr Pro Glu Val Thr Cys
            130                    135                    140

        Val Val Val Asp Val Ser Asp Glu Asp Pro Glu Val Lys Phe Asn Trp
        145                    150                    155                    160

        Tyr Val Asp Gly Val Glu Val His Asn Ala Lys Thr Lys Pro Arg Glu
                        165                    170                    175

        Glu Leu Tyr Asn Ser Thr Tyr Arg Val Val Ser Val Leu Thr Val Leu
                        180                    185                    190

        His Arg Asp Trp Leu Asn Gly Lys Glu Tyr Lys Cys Lys Val Ser Asn
                        195                    200                    205

        Thr Ala Leu Pro Lys Pro Ile Glu Lys Thr Ile Ser Lys Ala Lys Gly
            210                    215                    220

        Gln Arg Arg Glu Pro Gln Val Tyr Thr Leu Pro Pro Ser Arg Glu Glu
        225                    230                    235                    240

        Met Thr Lys Asn Gln Val Ser Leu Thr Cys Leu Val Lys Gly Phe Tyr
                        245                    250                    255

        Pro Ser Asp Ile Ala Val Glu Trp Glu Ser Asn Gly Gln Pro Glu Asn
                        260                    265                    270

        Asn Tyr Lys Thr Thr Pro Pro Val Leu Asp Ser Asp Gly Ser Phe Phe
                        275                    280                    285

        Leu Tyr Ser Lys Leu Thr Val Asp Lys Ser Arg Trp Gln Gln Gly Asn
            290                    295                    300

        Val Phe Ser Cys Ser Val Leu His Glu Ala Leu His Ala His Thr Thr
        305                    310                    315                    320

        Arg Lys Glu Leu Ser Leu Ser Pro
                        325

        <210>   14
        <211>   328
        <212>   PRT
        <213>   Artificial Sequence

        <220>
        <223>   IgG heavy chain constant region SG1080

<400> 14

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
            35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Asn Gly Pro Ser Val Phe Leu Phe Pro Pro
            115                 120                 125

Lys Pro Lys Asp Thr Leu Met Ile Ser Arg Thr Pro Glu Val Thr Cys
        130                 135                 140

Val Val Val Asp Val Ser Asp Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Leu Tyr Asn Ser Thr Tyr Arg Val Val Ser Val Leu Thr Val Leu
            180                 185                 190

His Arg Asp Trp Leu Asn Gly Lys Glu Tyr Lys Cys Lys Val Ser Asn
        195                 200                 205

Thr Ala Leu Pro Lys Pro Ile Glu Lys Thr Ile Ser Lys Ala Lys Gly
        210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
```

                    245                    250                    255

Pro Ser Asp Ile Ala Val Glu Trp Glu Ser Asn Gly Gln Pro Glu Asn
            260                265                270

Asn Tyr Lys Thr Thr Pro Pro Val Leu Asp Ser Asp Gly Ser Phe Phe
        275                280                285

Leu Tyr Ser Lys Leu Thr Val Asp Lys Ser Arg Trp Gln Gln Gly Asn
    290                295                300

Val Phe Ser Cys Ser Val Met His Glu Ala Leu His Ala His Tyr Thr
305                310                315                320

Arg Lys Glu Leu Ser Leu Ser Pro
                325

<210> 15
<211> 328
<212> PRT
<213> Artificial Sequence

<220>
<223> IgG heavy chain constant region SG1081

<400> 15

Ala Ser Thr Lys Gly Pro Ser Val Phe Pro Leu Ala Pro Ser Ser Lys
1               5                10               15

Ser Thr Ser Gly Gly Thr Ala Ala Leu Gly Cys Leu Val Lys Asp Tyr
        20                25                30

Phe Pro Glu Pro Val Thr Val Ser Trp Asn Ser Gly Ala Leu Thr Ser
        35                40                45

Gly Val His Thr Phe Pro Ala Val Leu Gln Ser Ser Gly Leu Tyr Ser
    50                55                60

Leu Ser Ser Val Val Thr Val Pro Ser Ser Ser Leu Gly Thr Gln Thr
65                70                75                80

Tyr Ile Cys Asn Val Asn His Lys Pro Ser Asn Thr Lys Val Asp Lys
                85                90                95

Lys Val Glu Pro Lys Ser Cys Asp Lys Thr His Thr Cys Pro Pro Cys
            100                105                110

Pro Ala Pro Glu Leu Trp Asn Gly Pro Ser Val Phe Leu Phe Pro Pro
        115                120                125

```
Lys Pro Lys Asp Thr Leu Met Ile Ser Arg Thr Pro Glu Val Thr Cys
    130                 135                 140

Val Val Val Asp Val Ser Asp Glu Asp Pro Glu Val Lys Phe Asn Trp
    145                 150                 155                 160

Tyr Val Asp Gly Val Glu Val His Asn Ala Lys Thr Lys Pro Arg Glu
                165                 170                 175

Glu Leu Tyr Asn Ser Thr Tyr Arg Val Val Ser Val Leu Thr Val Leu
                180                 185                 190

His Arg Asp Trp Leu Asn Gly Lys Glu Tyr Lys Cys Lys Val Ser Asn
        195                 200                 205

Thr Ala Leu Pro Lys Pro Ile Glu Lys Thr Ile Ser Lys Ala Lys Gly
    210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Ala His Tyr Thr
305                 310                 315                 320

Arg Lys Glu Leu Ser Leu Ser Pro
                325
```

```
<210>  16
<211>  328
<212>  PRT
<213>  Artificial Sequence

<220>
<223>  IgG heavy chain constant region PK55

<400>  16
```

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Asn Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser Asp Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
        180                 185                 190

His Arg Asp Trp Leu Asn Gly Lys Glu Tyr Lys Cys Lys Val Ser Asn
        195                 200                 205

Lys Ala Leu Pro Lys Pro Ile Glu Lys Thr Ile Ser Lys Ala Lys Gly
    210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255
```

33

```
Pro Ser Asp Ile Ala Val Glu Trp Glu Ser Asn Gly Gln Pro Glu Asn
        260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
        290             295             300

Val Phe Ser Cys Ser Val Met His Glu Ala Leu His Asn His Tyr Thr
305             310             315             320

Gln Lys Ser Leu Ser Leu Ser Pro
                325
```

```
<210>  17
<211>  328
<212>  PRT
<213>  Artificial Sequence

<220>
<223>  IgG heavy chain constant region PK56

<400>  17

Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
        20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
    50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90              95

Lys Val Glu Pro Lys Ser Cys Asp Lys Thr His Thr Cys Pro Pro Cys
        100             105             110

Pro Ala Pro Glu Leu Leu Asn Gly Pro Ser Val Phe Leu Phe Pro Pro
        115             120             125
```

```
Lys Pro Lys Asp Thr Leu Met Ile Ser Arg Thr Pro Glu Val Thr Cys
    130             135             140

Val Val Val Asp Val Ser Asp Glu Asp Pro Glu Val Lys Phe Asn Trp
    145             150             155             160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Thr Val Leu
            180             185             190

His Arg Asp Trp Leu Asn Gly Lys Glu Tyr Lys Cys Lys Val Ser Asn
        195             200             205

Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
        260             265             270

Asn Tyr Lys Thr Thr Pro Pro Val Leu Asp Ser Asp Gly Ser Phe Phe
        275             280             285

Leu Tyr Ser Lys Leu Thr Val Arg Lys Ser Arg Trp Gln Gln Gly Asn
    290             295             300

Val Phe Ser Cys Ser Val Met His Glu Ala Leu His Asn His Tyr Thr
305             310             315             320

Gln Lys Ser Leu Ser Leu Ser Pro
                325
```

```
<210>  18
<211>  328
<212>  PRT
<213>  Artificial Sequence

<220>
<223>  IgG heavy chain constant region TT20

<400>  18
```

```
Ala Ser Thr Lys Gly Pro Ser Val Phe Pro Leu Ala Pro Ser Ser Lys
1               5               10                  15

Ser Thr Ser Gly Gly Thr Ala Ala Leu Gly Cys Leu Val Lys Asp Tyr
            20              25                  30

Phe Pro Glu Pro Val Thr Val Ser Trp Asn Ser Gly Ala Leu Thr Ser
        35              40                  45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75                  80

Tyr Ile Cys Asn Val Asn His Lys Pro Ser Asn Thr Lys Val Asp Lys
            85              90                  95

Lys Val Glu Pro Lys Ser Cys Asp Lys Thr His Thr Cys Pro Pro Cys
            100             105                 110

Pro Ala Pro Glu Leu Leu Gly Gly Asp Ser Val Phe Leu Phe Pro Pro
        115             120             125

Lys Pro Lys Asp Val Leu Met Ile Ser Arg Thr Pro Glu Val Thr Cys
    130             135                 140

Val Val Val Asp Val Ser His Glu Asp Pro Glu Val Lys Phe Asn Trp
145             150             155                 160

Tyr Val Asp Gly Val Glu Val His Asn Ala Lys Thr Lys Pro Arg Glu
            165             170                 175

Glu Gln Tyr Asn Ser Thr Tyr Arg Val Val Ser Val Leu Pro Val Leu
        180             185             190

His Arg Asp Trp Leu Asn Gly Lys Glu Tyr Lys Cys Lys Val Ser Asn
        195             200             205

Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235                 240

Met Thr Lys Asn Gln Val Ser Leu Thr Cys Leu Val Lys Gly Phe Tyr
            245             250                 255
```

```
Pro Ser Asp Ile Ala Val Glu Trp Glu Ser Asn Gly Gln Pro Glu Asn
        260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
        290             295             300

Val Phe Ser Cys Ser Val Met His Glu Ala Leu His Asn His Tyr Thr
305             310             315             320

Gln Lys Ser Leu Ser Leu Ser Pro
                325
```

```
<210>   19
<211>   328
<212>   PRT
<213>   Artificial Sequence

<220>
<223>   IgG heavy chain constant region TT22

<400>   19
```

```
Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
        20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90              95

Lys Val Glu Pro Lys Ser Cys Asp Lys Thr His Thr Cys Pro Pro Cys
        100             105             110

Pro Ala Pro Glu Leu Leu Gly Gly Asp Ser Val Phe Leu Phe Pro Pro
        115             120             125
```

37

```
Lys Pro Lys Asp Val Leu Met Ile Ser Arg Thr Pro Glu Val Thr Cys
    130                 135                 140

Val Val Ile Asp Val Ser His Glu Asp Pro Glu Val Lys Phe Asn Trp
    145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
            180                 185                 190

His Arg Asp Trp Leu Asn Gly Lys Glu Tyr Lys Cys Lys Val Ser Asn
            195                 200                 205

Lys Ala Leu Pro Lys Pro Ile Glu Lys Thr Ile Ser Lys Ala Lys Gly
    210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325
```

```
<210>   20
<211>   328
<212>   PRT
<213>   Artificial Sequence

<220>
<223>   IgG heavy chain constant region TT24

<400>   20

Ala Ser Thr Lys Gly Pro Ser Val Phe Pro Leu Ala Pro Ser Ser Lys
```

|  | 1 | | | | 5 | | | | | 10 | | | | | 15 |
|---|---|---|---|---|---|---|---|---|---|---|---|---|---|---|---|

Ser Thr Ser Gly Gly Thr Ala Ala Leu Gly Cys Leu Val Lys Asp Tyr
        20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
        100             105             110

Pro Ala Pro Glu Leu Leu Gly Gly Asp Ser Val Phe Leu Phe Pro Pro
        115             120             125

Lys Pro Lys Asp Val Leu Met Ile Ser Arg Thr Pro Glu Val Thr Cys
        130             135             140

Val Val Val Asp Val Ala His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Pro Val Leu
            180             185             190

His Arg Asp Trp Leu Asn Gly Lys Glu Tyr Lys Cys Lys Val Ser Asn
        195             200             205

Lys Ala Leu Pro Lys Pro Ile Glu Lys Thr Ile Ser Lys Ala Lys Gly
        210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

```
            Pro Ser Asp Ile Ala Val Glu Trp Glu Ser Asn Gly Gln Pro Glu Asn
                    260                 265                 270


            Asn Tyr Lys Thr Thr Pro Pro Val Leu Asp Ser Asp Gly Ser Phe Phe
                    275                 280                 285


            Leu Tyr Ser Lys Leu Thr Val Asp Lys Ser Arg Trp Gln Gln Gly Asn
                    290                 295                 300


            Val Phe Ser Cys Ser Val Met His Glu Ala Leu His Asn His Tyr Thr
            305                 310                 315                 320


            Gln Lys Ser Leu Ser Leu Ser Pro
                                325


            <210>  21
            <211>  328
            <212>  PRT
            <213>  Artificial Sequence

            <220>
            <223>  IgG heavy chain constant region TT26

            <400>  21

            Ala Ser Thr Lys Gly Pro Ser Val Phe Pro Leu Ala Pro Ser Ser Lys
            1                   5                   10                  15


            Ser Thr Ser Gly Gly Thr Ala Ala Leu Gly Cys Leu Val Lys Asp Tyr
                    20                  25                  30


            Phe Pro Glu Pro Val Thr Val Ser Trp Asn Ser Gly Ala Leu Thr Ser
                    35                  40                  45


            Gly Val His Thr Phe Pro Ala Val Leu Gln Ser Ser Gly Leu Tyr Ser
                    50                  55                  60


            Leu Ser Ser Val Val Thr Val Pro Ser Ser Ser Leu Gly Thr Gln Thr
            65                  70                  75                  80


            Tyr Ile Cys Asn Val Asn His Lys Pro Ser Asn Thr Lys Val Asp Lys
                                85                  90                  95


            Lys Val Glu Pro Lys Ser Cys Asp Lys Thr His Thr Cys Pro Pro Cys
                        100                 105                 110


            Pro Ala Pro Glu Tyr Leu Gly Gly Asp Ser Val Phe Leu Phe Pro Pro
                        115                 120                 125


            Lys Pro Lys Asp Val Leu Met Ile Ser Arg Thr Pro Glu Val Thr Cys
```

```
                130                      135                           140


        Val Val Val Asp Val Ser His Glu Asp Pro Glu Val Lys Phe Asn Trp
        145             150             155                         160


        Tyr Val Asp Gly Val Glu Val His Asn Ala Lys Thr Lys Pro Arg Glu
                    165             170                 175


        Glu Gln Tyr Asn Ser Thr Tyr Arg Val Val Ser Val Leu Pro Val Leu
                    180             185                 190


        His Arg Asp Trp Leu Asn Gly Lys Glu Tyr Lys Cys Lys Val Ser Asn
                    195             200                 205


        Lys Ala Leu Pro Lys Pro Ile Glu Lys Thr Ile Ser Lys Ala Lys Gly
            210             215                 220


        Gln Arg Arg Glu Pro Gln Val Tyr Thr Leu Pro Pro Ser Arg Glu Glu
        225             230                 235                     240


        Met Thr Lys Asn Gln Val Ser Leu Thr Cys Leu Val Lys Gly Phe Tyr
                    245             250                 255


        Pro Ser Asp Ile Ala Val Glu Trp Glu Ser Asn Gly Gln Pro Glu Asn
                    260             265                 270


        Asn Tyr Lys Thr Thr Pro Pro Val Leu Asp Ser Asp Gly Ser Phe Phe
                    275             280                 285


        Leu Tyr Ser Lys Leu Thr Val Asp Lys Ser Arg Trp Gln Gln Gly Asn
            290             295                 300


        Val Phe Ser Cys Ser Val Met His Glu Ala Leu His Asn His Tyr Thr
        305             310                 315                     320


        Gln Lys Ser Leu Ser Leu Ser Pro
                    325
```

```
<210>   22
<211>   328
<212>   PRT
<213>   Artificial Sequence

<220>
<223>   IgG heavy chain constant region TT28

<400>   22

Ala Ser Thr Lys Gly Pro Ser Val Phe Pro Leu Ala Pro Ser Ser Lys
1               5                   10                  15
```

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
        100             105             110

Pro Ala Pro Glu Tyr Leu Gly Gly Asp Ser Val Phe Leu Phe Pro Pro
        115             120             125

Lys Pro Lys Asp Val Leu Met Ile Ser Arg Thr Pro Glu Val Thr Cys
        130             135             140

Val Val Val Asp Val Ala His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Pro Val Leu
        180             185             190

His Arg Asp Trp Leu Asn Gly Lys Glu Tyr Lys Cys Lys Val Ser Asn
        195             200             205

Lys Ala Leu Pro Lys Pro Ile Glu Lys Thr Ile Ser Lys Ala Lys Gly
        210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn

```
                 260                        265                        270

        Asn Tyr Lys Thr Thr Pro Pro Val Leu Asp Ser Asp Gly Ser Phe Phe
                275                 280                 285

        Leu Tyr Ser Lys Leu Thr Val Asp Lys Ser Arg Trp Gln Gln Gly Asn
                290                 295                 300

        Val Phe Ser Cys Ser Val Met His Glu Ala Leu His Asn His Tyr Thr
        305                 310                 315                 320

        Gln Lys Ser Leu Ser Leu Ser Pro
                        325


        <210>  23
        <211>  328
        <212>  PRT
        <213>  Artificial Sequence

        <220>
        <223>  IgG heavy chain constant region TT30

        <400>  23

        Ala Ser Thr Lys Gly Pro Ser Val Phe Pro Leu Ala Pro Ser Ser Lys
        1                   5                   10                  15

        Ser Thr Ser Gly Gly Thr Ala Ala Leu Gly Cys Leu Val Lys Asp Tyr
                        20                  25                  30

        Phe Pro Glu Pro Val Thr Val Ser Trp Asn Ser Gly Ala Leu Thr Ser
                        35                  40                  45

        Gly Val His Thr Phe Pro Ala Val Leu Gln Ser Ser Gly Leu Tyr Ser
                50                  55                  60

        Leu Ser Ser Val Val Thr Val Pro Ser Ser Ser Leu Gly Thr Gln Thr
        65                  70                  75                  80

        Tyr Ile Cys Asn Val Asn His Lys Pro Ser Asn Thr Lys Val Asp Lys
                        85                  90                  95

        Lys Val Glu Pro Lys Ser Cys Asp Lys Thr His Thr Cys Pro Pro Cys
                        100                 105                 110

        Pro Ala Pro Glu Leu Leu Gly Gly Asp Ser Val Phe Leu Phe Pro Pro
                        115                 120                 125

        Lys Pro Lys Asp Val Leu Met Ile Ser Arg Thr Pro Glu Val Thr Cys
                130                 135                 140
```

```
Val Val Ile Asp Val Ala His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Pro Val Leu
            180             185             190

His Arg Asp Trp Leu Asn Gly Lys Glu Tyr Lys Cys Lys Val Ser Asn
            195             200             205

Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
            275             280             285

Leu Tyr Ser Lys Leu Thr Val Asp Lys Ser Arg Trp Gln Gln Gly Asn
    290             295             300

Val Phe Ser Cys Ser Val Met His Glu Ala Leu His Asn His Tyr Thr
305             310             315             320

Gln Lys Ser Leu Ser Leu Ser Pro
            325
```

```
<210>  24
<211>  328
<212>  PRT
<213>  Artificial Sequence

<220>
<223>  IgG heavy chain constant region TT32

<400>  24

Ala Ser Thr Lys Gly Pro Ser Val Phe Pro Leu Ala Pro Ser Ser Lys
1               5               10              15
```

Ser Thr Ser Gly Gly Thr Ala Ala Leu Gly Cys Leu Val Lys Asp Tyr
        20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90              95

Lys Val Glu Pro Lys Ser Cys Asp Lys Thr His Thr Cys Pro Pro Cys
        100             105             110

Pro Ala Pro Glu Tyr Leu Gly Gly Asp Ser Val Phe Leu Phe Pro Pro
        115             120             125

Lys Pro Lys Asp Val Leu Met Ile Ser Arg Thr Pro Glu Val Thr Cys
        130             135             140

Val Val Ile Asp Val Ser His Glu Asp Pro Glu Val Lys Phe Asn Trp
145             150             155             160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Pro Val Leu
                180             185             190

His Arg Asp Trp Leu Asn Gly Lys Glu Tyr Lys Cys Lys Val Ser Asn
        195             200             205

Lys Ala Leu Pro Lys Pro Ile Glu Lys Thr Ile Ser Lys Ala Lys Gly
        210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
                245             250             255

Pro Ser Asp Ile Ala Val Glu Trp Glu Ser Asn Gly Gln Pro Glu Asn
        260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
        290             295             300

Val Phe Ser Cys Ser Val Met His Glu Ala Leu His Asn His Tyr Thr
305             310             315             320

Gln Lys Ser Leu Ser Leu Ser Pro
                325

<210>  25
<211>  328
<212>  PRT
<213>  Artificial Sequence

<220>
<223>  IgG heavy chain constant region TT34

<400>  25

Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
        20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90              95

Lys Val Glu Pro Lys Ser Cys Asp Lys Thr His Thr Cys Pro Pro Cys
            100             105             110

Pro Ala Pro Glu Tyr Leu Gly Gly Asp Ser Val Phe Leu Phe Pro Pro
        115             120             125

Lys Pro Lys Asp Val Leu Met Ile Ser Arg Thr Pro Glu Val Thr Cys
        130             135             140

```
Val Val Ile Asp Val Ala His Glu Asp Pro Glu Val Lys Phe Asn Trp
145             150             155             160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Pro Val Leu
            180             185             190

His Arg Asp Trp Leu Asn Gly Lys Glu Tyr Lys Cys Lys Val Ser Asn
            195             200             205

Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
            275             280             285

Leu Tyr Ser Lys Leu Thr Val Asp Lys Ser Arg Trp Gln Gln Gly Asn
    290             295             300

Val Phe Ser Cys Ser Val Met His Glu Ala Leu His Asn His Tyr Thr
305             310             315             320

Gln Lys Ser Leu Ser Leu Ser Pro
                325
```

```
<210>   26
<211>   328
<212>   PRT
<213>   Artificial Sequence

<220>
<223>   IgG heavy chain constant region TT66

<400>   26

Ala Ser Thr Lys Gly Pro Ser Val Phe Pro Leu Ala Pro Ser Ser Lys
1               5               10              15
```

Ser Thr Ser Gly Gly Thr Ala Ala Leu Gly Cys Leu Val Lys Asp Tyr
                20              25                  30

Phe Pro Glu Pro Val Thr Val Ser Trp Asn Ser Gly Ala Leu Thr Ser
            35              40                  45

Gly Val His Thr Phe Pro Ala Val Leu Gln Ser Ser Gly Leu Tyr Ser
        50              55                  60

Leu Ser Ser Val Val Thr Val Pro Ser Ser Ser Leu Gly Thr Gln Thr
65              70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100             105                 110

Pro Ala Pro Glu Leu Leu Gly Gly Asp Ser Val Phe Leu Phe Pro Pro
        115             120                 125

Lys Pro Lys Asp Val Leu Met Ile Ser Arg Thr Pro Glu Val Thr Cys
        130             135                 140

Val Val Val Asp Val Ser His Glu Asp Pro Glu Val Lys Phe Asn Trp
145             150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
            180             185                 190

His Arg Asp Trp Leu Asn Gly Lys Glu Tyr Lys Cys Lys Val Ser Asn
        195             200                 205

Lys Ala Leu Pro Lys Pro Ile Glu Lys Thr Ile Ser Lys Ala Lys Gly
        210             215                 220

Gln Arg Arg Glu Pro Gln Val Tyr Thr Leu Pro Pro Ser Arg Glu Glu
225             230                 235                 240

Met Thr Lys Asn Gln Val Ser Leu Thr Cys Leu Val Lys Gly Phe Tyr
            245             250                 255

Pro Ser Asp Ile Ala Val Glu Trp Glu Ser Asn Gly Gln Pro Glu Asn
            260             265                 270

48

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
        290             295             300

Val Phe Ser Cys Ser Val Met His Glu Ala Leu His Ala His Tyr Thr
305             310             315             320

Arg Lys Glu Leu Ser Leu Ser Pro
                325

<210>   27
<211>   328
<212>   PRT
<213>   Artificial Sequence

<220>
<223>   IgG heavy chain constant region TT70

<400>   27

Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
    50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
        100             105             110

Pro Ala Pro Glu Leu Leu Gly Gly Asp Ser Val Phe Leu Phe Pro Pro
        115             120             125

Lys Pro Lys Asp Val Leu Met Ile Ser Arg Thr Pro Glu Val Thr Cys
    130             135             140

```
Val Val Ile Asp Val Ser His Glu Asp Pro Glu Val Lys Phe Asn Trp
145             150             155             160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Pro Val Leu
            180             185             190

His Arg Asp Trp Leu Asn Gly Lys Glu Tyr Lys Cys Lys Val Ser Asn
            195             200             205

Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
            275             280             285

Leu Tyr Ser Lys Leu Thr Val Asp Lys Ser Arg Trp Gln Gln Gly Asn
    290             295             300

Val Phe Ser Cys Ser Val Met His Glu Ala Leu His Ala His Tyr Thr
305             310             315             320

Arg Lys Glu Leu Ser Leu Ser Pro
                325
```

```
<210>   28
<211>   328
<212>   PRT
<213>   Artificial Sequence

<220>
<223>   IgG heavy chain constant region

<400>   28
```

```
Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
```

|  | | | | 20 | | | | | 25 | | | | | 30 | | |
|---|---|---|---|---|---|---|---|---|---|---|---|---|---|---|---|---|

Phe Pro Glu Pro Val Thr Val Ser Trp Asn Ser Gly Ala Leu Thr Ser
     35             40             45

Gly Val His Thr Phe Pro Ala Val Leu Gln Ser Ser Gly Leu Tyr Ser
     50             55             60

Leu Ser Ser Val Val Thr Val Pro Ser Ser Ser Leu Gly Thr Gln Thr
65              70          75             80

Tyr Ile Cys Asn Val Asn His Lys Pro Ser Asn Thr Lys Val Asp Lys
         85         90             95

Lys Val Glu Pro Lys Ser Cys Asp Lys Thr His Thr Cys Pro Pro Cys
     100         105         110

Pro Ala Pro Glu Leu Leu Gly Gly Asp Ser Val Phe Leu Phe Pro Pro
     115         120         125

Lys Pro Lys Asp Val Leu Met Ile Ser Arg Thr Pro Glu Val Thr Cys
     130         135         140

Val Val Val Asp Val Ala His Glu Asp Pro Glu Val Lys Phe Asn Trp
145             150         155            160

Tyr Val Asp Gly Val Glu Val His Asn Ala Lys Thr Lys Pro Arg Glu
         165         170         175

Glu Gln Tyr Asn Ser Thr Tyr Arg Val Val Ser Val Leu Pro Val Leu
         180         185         190

His Arg Asp Trp Leu Asn Gly Lys Glu Tyr Lys Cys Lys Val Ser Asn
     195         200         205

Lys Ala Leu Pro Lys Pro Ile Glu Lys Thr Ile Ser Lys Ala Lys Gly
     210         215         220

Gln Arg Arg Glu Pro Gln Val Tyr Thr Leu Pro Pro Ser Arg Glu Glu
225             230         235            240

Met Thr Lys Asn Gln Val Ser Leu Thr Cys Leu Val Lys Gly Phe Tyr
         245         250         255

Pro Ser Asp Ile Ala Val Glu Trp Glu Ser Asn Gly Gln Pro Glu Asn
     260         265         270

```
Asn Tyr Lys Thr Thr Pro Pro Val Leu Asp Ser Asp Gly Ser Phe Phe
        275                 280             285

Leu Tyr Ser Lys Leu Thr Val Asp Lys Ser Arg Trp Gln Gln Gly Asn
        290                 295             300

Val Phe Ser Cys Ser Val Met His Glu Ala Leu His Ala His Tyr Thr
305             310             315                 320

Arg Lys Glu Leu Ser Leu Ser Pro
                325


<210>  29
<211>  328
<212>  PRT
<213>  Artificial Sequence

<220>
<223>  IgG heavy chain constant region

<400>  29

Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
        20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90                  95

Lys Val Glu Pro Lys Ser Cys Asp Lys Thr His Thr Cys Pro Pro Cys
            100             105             110

Pro Ala Pro Glu Tyr Leu Gly Gly Asp Ser Val Phe Leu Phe Pro Pro
            115             120             125

Lys Pro Lys Asp Val Leu Met Ile Ser Arg Thr Pro Glu Val Thr Cys
        130             135             140

Val Val Val Asp Val Ser His Glu Asp Pro Glu Val Lys Phe Asn Trp
```

52

<pre>
        145                    150                    155                    160


        Tyr Val Asp Gly Val Glu Val His Asn Ala Lys Thr Lys Pro Arg Glu
                        165                170                175


        Glu Gln Tyr Asn Ser Thr Tyr Arg Val Val Ser Val Leu Pro Val Leu
                        180                185                190


        His Arg Asp Trp Leu Asn Gly Lys Glu Tyr Lys Cys Lys Val Ser Asn
                        195                200                205


        Lys Ala Leu Pro Lys Pro Ile Glu Lys Thr Ile Ser Lys Ala Lys Gly
                210                215                220


        Gln Arg Arg Glu Pro Gln Val Tyr Thr Leu Pro Pro Ser Arg Glu Glu
        225                230                235                240


        Met Thr Lys Asn Gln Val Ser Leu Thr Cys Leu Val Lys Gly Phe Tyr
                        245                250                255


        Pro Ser Asp Ile Ala Val Glu Trp Glu Ser Asn Gly Gln Pro Glu Asn
                        260                265                270


        Asn Tyr Lys Thr Thr Pro Pro Val Leu Asp Ser Asp Gly Ser Phe Phe
                        275                280                285


        Leu Tyr Ser Lys Leu Thr Val Asp Lys Ser Arg Trp Gln Gln Gly Asn
                290                295                300


        Val Phe Ser Cys Ser Val Met His Glu Ala Leu His Ala His Tyr Thr
        305                310                315                320


        Arg Lys Glu Leu Ser Leu Ser Pro
                        325


        <210>   30
        <211>   328
        <212>   PRT
        <213>   Artificial Sequence

        <220>
        <223>   IgG heavy chain constant region

        <400>   30

        Ala Ser Thr Lys Gly Pro Ser Val Phe Pro Leu Ala Pro Ser Ser Lys
        1                5                  10                15


        Ser Thr Ser Gly Gly Thr Ala Ala Leu Gly Cys Leu Val Lys Asp Tyr
                        20                 25                 30
</pre>

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Tyr Leu Gly Gly Asp Ser Val Phe Leu Phe Pro Pro
            115                 120                 125

Lys Pro Lys Asp Val Leu Met Ile Ser Arg Thr Pro Glu Val Thr Cys
        130                 135                 140

Val Val Val Asp Val Ala His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
            180                 185                 190

His Arg Asp Trp Leu Asn Gly Lys Glu Tyr Lys Cys Lys Val Ser Asn
        195                 200                 205

Lys Ala Leu Pro Lys Pro Ile Glu Lys Thr Ile Ser Lys Ala Lys Gly
        210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
                260                 265                 270

Asn Tyr Lys Thr Thr Pro Pro Val Leu Asp Ser Asp Gly Ser Phe Phe
```

275                    280                    285

Leu Tyr Ser Lys Leu Thr Val Asp Lys Ser Arg Trp Gln Gln Gly Asn
    290                    295                    300

Val Phe Ser Cys Ser Val Met His Glu Ala Leu His Ala His Tyr Thr
305                    310                    315                    320

Arg Lys Glu Leu Ser Leu Ser Pro
                    325

<210>   31
<211>   328
<212>   PRT
<213>   Artificial Sequence

<220>
<223>   IgG heavy chain constant region

<400>   31

Ala Ser Thr Lys Gly Pro Ser Val Phe Pro Leu Ala Pro Ser Ser Lys
1                   5                   10                      15

Ser Thr Ser Gly Gly Thr Ala Ala Leu Gly Cys Leu Val Lys Asp Tyr
                20                      25                      30

Phe Pro Glu Pro Val Thr Val Ser Trp Asn Ser Gly Ala Leu Thr Ser
        35                      40                      45

Gly Val His Thr Phe Pro Ala Val Leu Gln Ser Ser Gly Leu Tyr Ser
        50                      55                      60

Leu Ser Ser Val Val Thr Val Pro Ser Ser Ser Leu Gly Thr Gln Thr
65                      70                      75                      80

Tyr Ile Cys Asn Val Asn His Lys Pro Ser Asn Thr Lys Val Asp Lys
                85                      90                      95

Lys Val Glu Pro Lys Ser Cys Asp Lys Thr His Thr Cys Pro Pro Cys
            100                     105                     110

Pro Ala Pro Glu Leu Leu Gly Gly Asp Ser Val Phe Leu Phe Pro Pro
            115                     120                     125

Lys Pro Lys Asp Val Leu Met Ile Ser Arg Thr Pro Glu Val Thr Cys
            130                     135                     140

Val Val Ile Asp Val Ala His Glu Asp Pro Glu Val Lys Phe Asn Trp
145                     150                     155                     160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Pro Val Leu
                180             185             190

His Arg Asp Trp Leu Asn Gly Lys Glu Tyr Lys Cys Lys Val Ser Asn
            195             200             205

Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
                245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
    290             295             300

Val Phe Ser Cys Ser Val Met His Glu Ala Leu His Ala His Tyr Thr
305             310             315             320

Arg Lys Glu Leu Ser Leu Ser Pro
                325

<210> 32
<211> 328
<212> PRT
<213> Artificial Sequence

<220>
<223> IgG heavy chain constant region TT90

<400> 32

Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
35              40              45

Gly Val His Thr Phe Pro Ala Val Leu Gln Ser Ser Gly Leu Tyr Ser
50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
85              90              95

Lys Val Glu Pro Lys Ser Cys Asp Lys Thr His Thr Cys Pro Pro Cys
100             105             110

Pro Ala Pro Glu Tyr Leu Gly Gly Asp Ser Val Phe Leu Phe Pro Pro
115             120             125

Lys Pro Lys Asp Val Leu Met Ile Ser Arg Thr Pro Glu Val Thr Cys
130             135             140

Val Val Ile Asp Val Ser His Glu Asp Pro Glu Val Lys Phe Asn Trp
145             150             155             160

Tyr Val Asp Gly Val Glu Val His Asn Ala Lys Thr Lys Pro Arg Glu
165             170             175

Glu Gln Tyr Asn Ser Thr Tyr Arg Val Val Ser Val Leu Pro Val Leu
180             185             190

His Arg Asp Trp Leu Asn Gly Lys Glu Tyr Lys Cys Lys Val Ser Asn
195             200             205

Lys Ala Leu Pro Lys Pro Ile Glu Lys Thr Ile Ser Lys Ala Lys Gly
210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
245             250             255

Pro Ser Asp Ile Ala Val Glu Trp Glu Ser Asn Gly Gln Pro Glu Asn
260             265             270

Asn Tyr Lys Thr Thr Pro Pro Val Leu Asp Ser Asp Gly Ser Phe Phe
275             280             285

EP 3 954 696 A1

Leu Tyr Ser Lys Leu Thr Val Asp Lys Ser Arg Trp Gln Gln Gly Asn
290                 295                 300

Val Phe Ser Cys Ser Val Met His Glu Ala Leu His Ala His Tyr Thr
305                 310                 315                 320

Arg Lys Glu Leu Ser Leu Ser Pro
                325

<210>  33
<211>  328
<212>  PRT
<213>  Artificial Sequence

<220>
<223>  IgG heavy chain constant region

<400>  33

Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
    50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
        100             105             110

Pro Ala Pro Glu Tyr Leu Gly Gly Asp Ser Val Phe Leu Phe Pro Pro
    115             120             125

Lys Pro Lys Asp Val Leu Met Ile Ser Arg Thr Pro Glu Val Thr Cys
    130             135             140

Val Val Ile Asp Val Ala His Glu Asp Pro Glu Val Lys Phe Asn Trp
145             150             155             160

58

Tyr Val Asp Gly Val Glu Val His Asn Ala Lys Thr Lys Pro Arg Glu
165             170             175

Glu Gln Tyr Asn Ser Thr Tyr Arg Val Val Ser Val Leu Pro Val Leu
180             185             190

His Arg Asp Trp Leu Asn Gly Lys Glu Tyr Lys Cys Lys Val Ser Asn
195             200             205

Lys Ala Leu Pro Lys Pro Ile Glu Lys Thr Ile Ser Lys Ala Lys Gly
210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
245             250             255

Pro Ser Asp Ile Ala Val Glu Trp Glu Ser Asn Gly Gln Pro Glu Asn
260             265             270

Asn Tyr Lys Thr Thr Pro Pro Val Leu Asp Ser Asp Gly Ser Phe Phe
275             280             285

Leu Tyr Ser Lys Leu Thr Val Asp Lys Ser Arg Trp Gln Gln Gly Asn
290             295             300

Val Phe Ser Cys Ser Val Met His Glu Ala Leu His Ala His Tyr Thr
305             310             315             320

Arg Lys Glu Leu Ser Leu Ser Pro
325

<210> 34
<211> 328
<212> PRT
<213> Artificial Sequence

<220>
<223> IgG heavy chain constant region

<400> 34

Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
20              25              30

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Asp Ser Val Phe Leu Phe Pro Pro
            115                 120                 125

Lys Pro Lys Asp Val Leu Met Ile Ser Arg Thr Pro Glu Val Thr Cys
        130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
            180                 185                 190

His Arg Asp Trp Leu Asn Gly Lys Glu Tyr Lys Cys Lys Val Ser Asn
        195                 200                 205

Lys Ala Leu Pro Lys Pro Ile Glu Lys Thr Ile Ser Lys Ala Lys Gly
        210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285
```

60

```
Leu Tyr Ser Lys Leu Thr Val Asp Lys Ser Arg Trp Gln Gln Gly Asn
    290                 295                 300

Val Phe Ser Cys Ser Val Met His Glu Ala Leu His Ala His Thr Thr
305                 310                 315                 320

Arg Lys Glu Leu Ser Leu Ser Pro
                325
```

<210> 35
<211> 328
<212> PRT
<213> Artificial Sequence

<220>
<223> IgG heavy chain constant region

<400> 35

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Asp Ser Val Phe Leu Phe Pro Pro
        115                 120                 125

Lys Pro Lys Asp Val Leu Met Ile Ser Arg Thr Pro Glu Val Thr Cys
    130                 135                 140

Val Val Ile Asp Val Ser His Glu Asp Pro Glu Val Lys Phe Asn Trp
145                 150                 155                 160
```

61

```
Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Pro Val Leu
                180             185             190

His Arg Asp Trp Leu Asn Gly Lys Glu Tyr Lys Cys Lys Val Ser Asn
                195             200             205

Lys Ala Leu Pro Lys Pro Ile Glu Lys Thr Ile Ser Lys Ala Lys Gly
        210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
                245             250             255

Pro Ser Asp Ile Ala Val Glu Trp Glu Ser Asn Gly Gln Pro Glu Asn
                260             265             270

Asn Tyr Lys Thr Thr Pro Pro Val Leu Asp Ser Asp Gly Ser Phe Phe
                275             280             285

Leu Tyr Ser Lys Leu Thr Val Asp Lys Ser Arg Trp Gln Gln Gly Asn
        290             295             300

Val Phe Ser Cys Ser Val Met His Glu Ala Leu His Ala His Thr Thr
305             310             315             320

Arg Lys Glu Leu Ser Leu Ser Pro
                325
```

```
<210>   36
<211>   328
<212>   PRT
<213>   Artificial Sequence

<220>
<223>   IgG heavy chain constant region

<400>   36

Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
                20              25              30

Phe Pro Glu Pro Val Thr Val Ser Trp Asn Ser Gly Ala Leu Thr Ser
```

|  | 35 |  |  |  | 40 |  |  |  | 45 |  |  |
|---|---|---|---|---|---|---|---|---|---|---|---|

Gly Val His Thr Phe Pro Ala Val Leu Gln Ser Ser Gly Leu Tyr Ser
    50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
            100             105             110

Pro Ala Pro Glu Leu Leu Gly Gly Asp Ser Val Phe Leu Phe Pro Pro
            115             120             125

Lys Pro Lys Asp Val Leu Met Ile Ser Arg Thr Pro Glu Val Thr Cys
    130             135             140

Val Val Val Asp Val Ala His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Pro Val Leu
            180             185             190

His Arg Asp Trp Leu Asn Gly Lys Glu Tyr Lys Cys Lys Val Ser Asn
    195             200             205

Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
    275             280             285

Leu Tyr Ser Lys Leu Thr Val Asp Lys Ser Arg Trp Gln Gln Gly Asn
290                 295                 300

Val Phe Ser Cys Ser Val Met His Glu Ala Leu His Ala His Thr Thr
305                 310                 315                 320

Arg Lys Glu Leu Ser Leu Ser Pro
                325

<210> 37
<211> 328
<212> PRT
<213> Artificial Sequence

<220>
<223> IgG heavy chain constant region

<400> 37

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
                20                  25                  30

Phe Pro Glu Pro Val Thr Val Ser Trp Asn Ser Gly Ala Leu Thr Ser
                35                  40                  45

Gly Val His Thr Phe Pro Ala Val Leu Gln Ser Ser Gly Leu Tyr Ser
                50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                        85                  90                  95

Lys Val Glu Pro Lys Ser Cys Asp Lys Thr His Thr Cys Pro Pro Cys
                100                 105                 110

Pro Ala Pro Glu Tyr Leu Gly Gly Asp Ser Val Phe Leu Phe Pro Pro
                115                 120                 125

Lys Pro Lys Asp Val Leu Met Ile Ser Arg Thr Pro Glu Val Thr Cys
                130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu

64

```
                    165                    170                    175

        Glu Gln Tyr Asn Ser Thr Tyr Arg Val Val Ser Val Leu Pro Val Leu
                    180                    185                    190

        His Arg Asp Trp Leu Asn Gly Lys Glu Tyr Lys Cys Lys Val Ser Asn
                    195                    200                    205

        Lys Ala Leu Pro Lys Pro Ile Glu Lys Thr Ile Ser Lys Ala Lys Gly
                    210                    215                    220

        Gln Arg Arg Glu Pro Gln Val Tyr Thr Leu Pro Pro Ser Arg Glu Glu
        225                    230                    235                    240

        Met Thr Lys Asn Gln Val Ser Leu Thr Cys Leu Val Lys Gly Phe Tyr
                    245                    250                    255

        Pro Ser Asp Ile Ala Val Glu Trp Glu Ser Asn Gly Gln Pro Glu Asn
                    260                    265                    270

        Asn Tyr Lys Thr Thr Pro Pro Val Leu Asp Ser Asp Gly Ser Phe Phe
                    275                    280                    285

        Leu Tyr Ser Lys Leu Thr Val Asp Lys Ser Arg Trp Gln Gln Gly Asn
                    290                    295                    300

        Val Phe Ser Cys Ser Val Met His Glu Ala Leu His Ala His Thr Thr
        305                    310                    315                    320

        Arg Lys Glu Leu Ser Leu Ser Pro
                    325
```

```
        <210>   38
        <211>   328
        <212>   PRT
        <213>   Artificial Sequence

        <220>
        <223>   IgG heavy chain constant region

        <400>   38

        Ala Ser Thr Lys Gly Pro Ser Val Phe Pro Leu Ala Pro Ser Ser Lys
        1                   5                    10                   15

        Ser Thr Ser Gly Gly Thr Ala Ala Leu Gly Cys Leu Val Lys Asp Tyr
                    20                   25                   30

        Phe Pro Glu Pro Val Thr Val Ser Trp Asn Ser Gly Ala Leu Thr Ser
                    35                   40                   45
```

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Tyr Leu Gly Gly Asp Ser Val Phe Leu Phe Pro Pro
115 120 125

Lys Pro Lys Asp Val Leu Met Ile Ser Arg Thr Pro Glu Val Thr Cys
130 135 140

Val Val Val Asp Val Ala His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Pro Val Leu
180 185 190

His Arg Asp Trp Leu Asn Gly Lys Glu Tyr Lys Cys Lys Val Ser Asn
195 200 205

Lys Ala Leu Pro Lys Pro Ile Glu Lys Thr Ile Ser Lys Ala Lys Gly
210 215 220

Gln Arg Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn

```
                  290                     295                         300


         Val Phe Ser Cys Ser Val Met His Glu Ala Leu His Ala His Thr Thr
         305                 310                 315                 320


         Arg Lys Glu Leu Ser Leu Ser Pro
                         325


         <210>  39
         <211>  328
         <212>  PRT
         <213>  Artificial Sequence

         <220>
         <223>   IgG heavy chain constant region

         <400>  39

         Ala Ser Thr Lys Gly Pro Ser Val Phe Pro Leu Ala Pro Ser Ser Lys
         1                   5                   10                  15


         Ser Thr Ser Gly Gly Thr Ala Ala Leu Gly Cys Leu Val Lys Asp Tyr
                         20                  25                  30


         Phe Pro Glu Pro Val Thr Val Ser Trp Asn Ser Gly Ala Leu Thr Ser
                 35                  40                  45


         Gly Val His Thr Phe Pro Ala Val Leu Gln Ser Ser Gly Leu Tyr Ser
                 50                  55                  60


         Leu Ser Ser Val Val Thr Val Pro Ser Ser Ser Leu Gly Thr Gln Thr
         65                  70                  75                  80


         Tyr Ile Cys Asn Val Asn His Lys Pro Ser Asn Thr Lys Val Asp Lys
                         85                  90                  95


         Lys Val Glu Pro Lys Ser Cys Asp Lys Thr His Thr Cys Pro Pro Cys
                         100                 105                 110


         Pro Ala Pro Glu Leu Leu Gly Gly Asp Ser Val Phe Leu Phe Pro Pro
                 115                 120                 125


         Lys Pro Lys Asp Val Leu Met Ile Ser Arg Thr Pro Glu Val Thr Cys
                 130                 135                 140


         Val Val Ile Asp Val Ala His Glu Asp Pro Glu Val Lys Phe Asn Trp
         145                 150                 155                 160


         Tyr Val Asp Gly Val Glu Val His Asn Ala Lys Thr Lys Pro Arg Glu
                         165                 170                 175
```

67

```
Glu Gln Tyr Asn Ser Thr Tyr Arg Val Val Ser Val Leu Pro Val Leu
            180                 185                 190

His Arg Asp Trp Leu Asn Gly Lys Glu Tyr Lys Cys Lys Val Ser Asn
            195                 200                 205

Lys Ala Leu Pro Lys Pro Ile Glu Lys Thr Ile Ser Lys Ala Lys Gly
            210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
            275                 280                 285

Leu Tyr Ser Lys Leu Thr Val Asp Lys Ser Arg Trp Gln Gln Gly Asn
            290                 295                 300

Val Phe Ser Cys Ser Val Met His Glu Ala Leu His Ala His Thr Thr
305                 310                 315                 320

Arg Lys Glu Leu Ser Leu Ser Pro
                325
```

```
<210>   40
<211>   328
<212>   PRT
<213>   Artificial Sequence

<220>
<223>   IgG heavy chain constant region

<400>   40
```

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
            35                  40                  45
```

```
Gly Val His Thr Phe Pro Ala Val Leu Gln Ser Ser Gly Leu Tyr Ser
    50              55              60

Leu Ser Ser Val Val Thr Val Pro Ser Ser Ser Leu Gly Thr Gln Thr
    65              70              75              80

Tyr Ile Cys Asn Val Asn His Lys Pro Ser Asn Thr Lys Val Asp Lys
                85              90              95

Lys Val Glu Pro Lys Ser Cys Asp Lys Thr His Thr Cys Pro Pro Cys
            100             105             110

Pro Ala Pro Glu Tyr Leu Gly Gly Asp Ser Val Phe Leu Phe Pro Pro
        115             120             125

Lys Pro Lys Asp Val Leu Met Ile Ser Arg Thr Pro Glu Val Thr Cys
    130             135             140

Val Val Ile Asp Val Ser His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Pro Val Leu
        180             185             190

His Arg Asp Trp Leu Asn Gly Lys Glu Tyr Lys Cys Lys Val Ser Asn
        195             200             205

Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
    290             295             300
```

69

```
Val Phe Ser Cys Ser Val Met His Glu Ala Leu His Ala His Thr Thr
305                 310             315                 320


Arg Lys Glu Leu Ser Leu Ser Pro
                325



<210>   41
<211>   328
<212>   PRT
<213>   Artificial Sequence

<220>
<223>   IgG heavy chain constant region

<400>   41

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
            35                  40                  45


Gly Val His Thr Phe Pro Ala Val Leu Gln Ser Ser Gly Leu Tyr Ser
        50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
                85                  90                  95


Lys Val Glu Pro Lys Ser Cys Asp Lys Thr His Thr Cys Pro Pro Cys
            100                 105                 110


Pro Ala Pro Glu Tyr Leu Gly Gly Asp Ser Val Phe Leu Phe Pro Pro
            115                 120                 125


Lys Pro Lys Asp Val Leu Met Ile Ser Arg Thr Pro Glu Val Thr Cys
        130                 135                 140


Val Val Ile Asp Val Ala His Glu Asp Pro Glu Val Lys Phe Asn Trp
145                 150                 155                 160


Tyr Val Asp Gly Val Glu Val His Asn Ala Lys Thr Lys Pro Arg Glu
                165                 170                 175
```

70

```
Glu Gln Tyr Asn Ser Thr Tyr Arg Val Val Ser Val Leu Pro Val Leu
            180                 185                 190

His Arg Asp Trp Leu Asn Gly Lys Glu Tyr Lys Cys Lys Val Ser Asn
            195                 200                 205

Lys Ala Leu Pro Lys Pro Ile Glu Lys Thr Ile Ser Lys Ala Lys Gly
            210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
            275                 280                 285

Leu Tyr Ser Lys Leu Thr Val Asp Lys Ser Arg Trp Gln Gln Gly Asn
            290                 295                 300

Val Phe Ser Cys Ser Val Met His Glu Ala Leu His Ala His Thr Thr
305                 310                 315                 320

Arg Lys Glu Leu Ser Leu Ser Pro
                325
```

```
<210>   42
<211>   328
<212>   PRT
<213>   Artificial Sequence

<220>
<223>   IgG heavy chain constant region TT67

<400>   42
```

```
Ala Ser Thr Lys Gly Pro Ser Val Phe Pro Leu Ala Pro Ser Ser Lys
1                   5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
            35                  40                  45
```

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Asp Ser Val Phe Leu Phe Pro Pro
115 120 125

Lys Pro Lys Asp Val Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Pro Val Leu
180 185 190

His Arg Asp Trp Leu Asn Gly Lys Glu Tyr Lys Cys Lys Val Ser Asn
195 200 205

Lys Ala Leu Pro Lys Pro Ile Glu Lys Thr Ile Ser Lys Ala Lys Gly
210 215 220

Gln Arg Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

```
Val Phe Ser Cys Ser Val Leu His Glu Ala Leu His Ala His Thr Thr
305                 310             315                 320


Arg Lys Glu Leu Ser Leu Ser Pro
                325



<210>   43
<211>   328
<212>   PRT
<213>   Artificial Sequence

<220>
<223>   IgG heavy chain constant region TT71

<400>   43

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
        50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
                85                  90                  95


Lys Val Glu Pro Lys Ser Cys Asp Lys Thr His Thr Cys Pro Pro Cys
            100                 105                 110


Pro Ala Pro Glu Leu Leu Gly Gly Asp Ser Val Phe Leu Phe Pro Pro
            115                 120                 125


Lys Pro Lys Asp Val Leu Met Ile Ser Arg Thr Pro Glu Val Thr Cys
        130                 135                 140


Val Val Ile Asp Val Ser His Glu Asp Pro Glu Val Lys Phe Asn Trp
145                 150                 155                 160


Tyr Val Asp Gly Val Glu Val His Asn Ala Lys Thr Lys Pro Arg Glu
                165                 170                 175
```

73

```
Glu Gln Tyr Asn Ser Thr Tyr Arg Val Val Ser Val Leu Pro Val Leu
            180                 185                 190

His Arg Asp Trp Leu Asn Gly Lys Glu Tyr Lys Cys Lys Val Ser Asn
            195                 200                 205

Lys Ala Leu Pro Lys Pro Ile Glu Lys Thr Ile Ser Lys Ala Lys Gly
    210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Leu His Glu Ala Leu His Ala His Thr Thr
305                 310                 315                 320

Arg Lys Glu Leu Ser Leu Ser Pro
                325
```

<210> 44
<211> 328
<212> PRT
<213> Artificial Sequence

<220>
<223> IgG heavy chain constant region

<400> 44

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
```

```
              50                        55                        60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Asp Ser Val Phe Leu Phe Pro Pro
            115                 120                 125

Lys Pro Lys Asp Val Leu Met Ile Ser Arg Thr Pro Glu Val Thr Cys
            130                 135                 140

Val Val Val Asp Val Ala His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
            180                 185                 190

His Arg Asp Trp Leu Asn Gly Lys Glu Tyr Lys Cys Lys Val Ser Asn
            195                 200                 205

Lys Ala Leu Pro Lys Pro Ile Glu Lys Thr Ile Ser Lys Ala Lys Gly
            210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
            275                 280                 285

Leu Tyr Ser Lys Leu Thr Val Asp Lys Ser Arg Trp Gln Gln Gly Asn
            290                 295                 300
```

```
Val Phe Ser Cys Ser Val Leu His Glu Ala Leu His Ala His Thr Thr
305                 310                 315                 320


Arg Lys Glu Leu Ser Leu Ser Pro
                325


<210>   45
<211>   328
<212>   PRT
<213>   Artificial Sequence

<220>
<223>   IgG heavy chain constant region

<400>   45

Ala Ser Thr Lys Gly Pro Ser Val Phe Pro Leu Ala Pro Ser Ser Lys
1                 5                   10                  15


Ser Thr Ser Gly Gly Thr Ala Ala Leu Gly Cys Leu Val Lys Asp Tyr
                20                  25                  30


Phe Pro Glu Pro Val Thr Val Ser Trp Asn Ser Gly Ala Leu Thr Ser
            35                  40                  45


Gly Val His Thr Phe Pro Ala Val Leu Gln Ser Ser Gly Leu Tyr Ser
        50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
                85                  90                  95


Lys Val Glu Pro Lys Ser Cys Asp Lys Thr His Thr Cys Pro Pro Cys
            100                 105                 110


Pro Ala Pro Glu Tyr Leu Gly Gly Asp Ser Val Phe Leu Phe Pro Pro
            115                 120                 125


Lys Pro Lys Asp Val Leu Met Ile Ser Arg Thr Pro Glu Val Thr Cys
        130                 135                 140


Val Val Val Asp Val Ser His Glu Asp Pro Glu Val Lys Phe Asn Trp
145                 150                 155                 160


Tyr Val Asp Gly Val Glu Val His Asn Ala Lys Thr Lys Pro Arg Glu
                165                 170                 175


Glu Gln Tyr Asn Ser Thr Tyr Arg Val Val Ser Val Leu Pro Val Leu
```

180             185             190

His Arg Asp Trp Leu Asn Gly Lys Glu Tyr Lys Cys Lys Val Ser Asn
     195            200            205

Lys Ala Leu Pro Lys Pro Ile Glu Lys Thr Ile Ser Lys Ala Lys Gly
     210            215            220

Gln Arg Arg Glu Pro Gln Val Tyr Thr Leu Pro Pro Ser Arg Glu Glu
225            230            235            240

Met Thr Lys Asn Gln Val Ser Leu Thr Cys Leu Val Lys Gly Phe Tyr
           245            250            255

Pro Ser Asp Ile Ala Val Glu Trp Glu Ser Asn Gly Gln Pro Glu Asn
     260            265            270

Asn Tyr Lys Thr Thr Pro Pro Val Leu Asp Ser Asp Gly Ser Phe Phe
     275            280            285

Leu Tyr Ser Lys Leu Thr Val Asp Lys Ser Arg Trp Gln Gln Gly Asn
     290            295            300

Val Phe Ser Cys Ser Val Leu His Glu Ala Leu His Ala His Thr Thr
305            310            315            320

Arg Lys Glu Leu Ser Leu Ser Pro
           325

<210> 46
<211> 328
<212> PRT
<213> Artificial Sequence

<220>
<223> IgG heavy chain constant region

<400> 46

Ala Ser Thr Lys Gly Pro Ser Val Phe Pro Leu Ala Pro Ser Ser Lys
1            5            10            15

Ser Thr Ser Gly Gly Thr Ala Ala Leu Gly Cys Leu Val Lys Asp Tyr
     20            25            30

Phe Pro Glu Pro Val Thr Val Ser Trp Asn Ser Gly Ala Leu Thr Ser
     35            40            45

Gly Val His Thr Phe Pro Ala Val Leu Gln Ser Ser Gly Leu Tyr Ser
     50            55            60

```
Leu Ser Ser Val Val Thr Val Pro Ser Ser Ser Leu Gly Thr Gln Thr
65                  70              75                      80


Tyr Ile Cys Asn Val Asn His Lys Pro Ser Asn Thr Lys Val Asp Lys
                    85                  90                  95


Lys Val Glu Pro Lys Ser Cys Asp Lys Thr His Thr Cys Pro Pro Cys
                100                 105                 110


Pro Ala Pro Glu Tyr Leu Gly Gly Asp Ser Val Phe Leu Phe Pro Pro
                115                 120                 125


Lys Pro Lys Asp Val Leu Met Ile Ser Arg Thr Pro Glu Val Thr Cys
                130                 135                 140


Val Val Val Asp Val Ala His Glu Asp Pro Glu Val Lys Phe Asn Trp
145                 150                 155                 160


Tyr Val Asp Gly Val Glu Val His Asn Ala Lys Thr Lys Pro Arg Glu
                165                 170                 175


Glu Gln Tyr Asn Ser Thr Tyr Arg Val Val Ser Val Leu Pro Val Leu
                180                 185                 190


His Arg Asp Trp Leu Asn Gly Lys Glu Tyr Lys Cys Lys Val Ser Asn
                195                 200                 205


Lys Ala Leu Pro Lys Pro Ile Glu Lys Thr Ile Ser Lys Ala Lys Gly
                210                 215                 220


Gln Arg Arg Glu Pro Gln Val Tyr Thr Leu Pro Pro Ser Arg Glu Glu
225                 230                 235                 240


Met Thr Lys Asn Gln Val Ser Leu Thr Cys Leu Val Lys Gly Phe Tyr
                245                 250                 255


Pro Ser Asp Ile Ala Val Glu Trp Glu Ser Asn Gly Gln Pro Glu Asn
                260                 265                 270


Asn Tyr Lys Thr Thr Pro Pro Val Leu Asp Ser Asp Gly Ser Phe Phe
                275                 280                 285


Leu Tyr Ser Lys Leu Thr Val Asp Lys Ser Arg Trp Gln Gln Gly Asn
                290                 295                 300


Val Phe Ser Cys Ser Val Leu His Glu Ala Leu His Ala His Thr Thr
```

305 310 315 320

Arg Lys Glu Leu Ser Leu Ser Pro
                        325


<210> 47
<211> 328
<212> PRT
<213> Artificial Sequence

<220>
<223> IgG heavy chain constant region

<400> 47

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
        50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
                85                  90                  95


Lys Val Glu Pro Lys Ser Cys Asp Lys Thr His Thr Cys Pro Pro Cys
            100                 105                 110


Pro Ala Pro Glu Leu Leu Gly Gly Asp Ser Val Phe Leu Phe Pro Pro
            115                 120                 125


Lys Pro Lys Asp Val Leu Met Ile Ser Arg Thr Pro Glu Val Thr Cys
            130                 135                 140


Val Val Ile Asp Val Ala His Glu Asp Pro Glu Val Lys Phe Asn Trp
145                 150                 155                 160


Tyr Val Asp Gly Val Glu Val His Asn Ala Lys Thr Lys Pro Arg Glu
                165                 170                 175


Glu Gln Tyr Asn Ser Thr Tyr Arg Val Val Ser Val Leu Pro Val Leu
                180                 185                 190

His Arg Asp Trp Leu Asn Gly Lys Glu Tyr Lys Cys Lys Val Ser Asn
        195                 200             205

Lys Ala Leu Pro Lys Pro Ile Glu Lys Thr Ile Ser Lys Ala Lys Gly
        210             215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245             250                 255

Pro Ser Asp Ile Ala Val Glu Trp Glu Ser Asn Gly Gln Pro Glu Asn
            260             265                 270

Asn Tyr Lys Thr Thr Pro Pro Val Leu Asp Ser Asp Gly Ser Phe Phe
        275             280                 285

Leu Tyr Ser Lys Leu Thr Val Asp Lys Ser Arg Trp Gln Gln Gly Asn
        290             295                 300

Val Phe Ser Cys Ser Val Leu His Glu Ala Leu His Ala His Thr Thr
305             310                 315                 320

Arg Lys Glu Leu Ser Leu Ser Pro
                325

<210> 48
<211> 328
<212> PRT
<213> Artificial Sequence

<220>
<223> IgG heavy chain constant region TT91

<400> 48

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                  60

```
Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
            100             105             110

Pro Ala Pro Glu Tyr Leu Gly Gly Asp Ser Val Phe Leu Phe Pro Pro
            115             120             125

Lys Pro Lys Asp Val Leu Met Ile Ser Arg Thr Pro Glu Val Thr Cys
            130             135             140

Val Val Ile Asp Val Ser His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Pro Val Leu
            180             185             190

His Arg Asp Trp Leu Asn Gly Lys Glu Tyr Lys Cys Lys Val Ser Asn
            195             200             205

Lys Ala Leu Pro Lys Pro Ile Glu Lys Thr Ile Ser Lys Ala Lys Gly
            210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
            275             280             285

Leu Tyr Ser Lys Leu Thr Val Asp Lys Ser Arg Trp Gln Gln Gly Asn
            290             295             300

Val Phe Ser Cys Ser Val Leu His Glu Ala Leu His Ala His Thr Thr
305             310             315             320
```

```
Arg Lys Glu Leu Ser Leu Ser Pro
                325


<210>  49
<211>  328
<212>  PRT
<213>  Artificial Sequence

<220>
<223>  IgG heavy chain constant region

<400>  49

Ala Ser Thr Lys Gly Pro Ser Val Phe Pro Leu Ala Pro Ser Ser Lys
1                5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
                85                  90                  95


Lys Val Glu Pro Lys Ser Cys Asp Lys Thr His Thr Cys Pro Pro Cys
            100                 105                 110


Pro Ala Pro Glu Tyr Leu Gly Gly Asp Ser Val Phe Leu Phe Pro Pro
            115                 120                 125


Lys Pro Lys Asp Val Leu Met Ile Ser Arg Thr Pro Glu Val Thr Cys
    130                 135                 140


Val Val Ile Asp Val Ala His Glu Asp Pro Glu Val Lys Phe Asn Trp
145                 150                 155                 160


Tyr Val Asp Gly Val Glu Val His Asn Ala Lys Thr Lys Pro Arg Glu
                165                 170                 175


Glu Gln Tyr Asn Ser Thr Tyr Arg Val Val Ser Val Leu Pro Val Leu
                180                 185                 190
```

82

```
        His Arg Asp Trp Leu Asn Gly Lys Glu Tyr Lys Cys Lys Val Ser Asn
            195                 200             205

        Lys Ala Leu Pro Lys Pro Ile Glu Lys Thr Ile Ser Lys Ala Lys Gly
            210             215                 220

        Gln Arg Arg Glu Pro Gln Val Tyr Thr Leu Pro Pro Ser Arg Glu Glu
        225             230             235                 240

        Met Thr Lys Asn Gln Val Ser Leu Thr Cys Leu Val Lys Gly Phe Tyr
                    245             250                 255

        Pro Ser Asp Ile Ala Val Glu Trp Glu Ser Asn Gly Gln Pro Glu Asn
                    260             265             270

        Asn Tyr Lys Thr Thr Pro Pro Val Leu Asp Ser Asp Gly Ser Phe Phe
                275             280             285

        Leu Tyr Ser Lys Leu Thr Val Asp Lys Ser Arg Trp Gln Gln Gly Asn
            290             295             300

        Val Phe Ser Cys Ser Val Leu His Glu Ala Leu His Ala His Thr Thr
        305             310             315                 320

        Arg Lys Glu Leu Ser Leu Ser Pro
                        325


        <210>  50
        <211>  328
        <212>  PRT
        <213>  Artificial Sequence

        <220>
        <223>  IgG heavy chain constant region

        <400>  50

        Ala Ser Thr Lys Gly Pro Ser Val Phe Pro Leu Ala Pro Ser Ser Lys
        1               5                   10                  15

        Ser Thr Ser Gly Gly Thr Ala Ala Leu Gly Cys Leu Val Lys Asp Tyr
                    20              25                  30

        Phe Pro Glu Pro Val Thr Val Ser Trp Asn Ser Gly Ala Leu Thr Ser
                    35              40                  45

        Gly Val His Thr Phe Pro Ala Val Leu Gln Ser Ser Gly Leu Tyr Ser
                50              55                  60
```

```
Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
            85              90              95

Lys Val Glu Pro Lys Ser Cys Asp Lys Thr His Thr Cys Pro Pro Cys
        100             105             110

Pro Ala Pro Glu Leu Leu Gly Gly Asp Ser Val Phe Leu Phe Pro Pro
        115             120             125

Lys Pro Lys Asp Val Leu Met Ile Ser Arg Thr Pro Glu Val Thr Cys
        130             135             140

Val Val Val Asp Val Ser His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Pro Val Leu
            180             185             190

His Arg Asp Trp Leu Asn Gly Lys Glu Tyr Lys Cys Lys Val Ser Asn
            195             200             205

Lys Ala Leu Pro Lys Pro Ile Glu Lys Thr Ile Ser Lys Ala Lys Gly
        210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
        290             295             300

Val Phe Ser Cys Ser Val Leu His Glu Ala Leu His Ala His Tyr Thr
305             310             315             320
```

Arg Lys Glu Leu Ser Leu Ser Pro
                325


<210>  51
<211>  328
<212>  PRT
<213>  Artificial Sequence

<220>
<223>  IgG heavy chain constant region

<400>  51

Ala Ser Thr Lys Gly Pro Ser Val Phe Pro Leu Ala Pro Ser Ser Lys
1                   5                   10                  15


Ser Thr Ser Gly Gly Thr Ala Ala Leu Gly Cys Leu Val Lys Asp Tyr
                20                  25                  30


Phe Pro Glu Pro Val Thr Val Ser Trp Asn Ser Gly Ala Leu Thr Ser
            35                  40                  45


Gly Val His Thr Phe Pro Ala Val Leu Gln Ser Ser Gly Leu Tyr Ser
        50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
                85                  90                  95


Lys Val Glu Pro Lys Ser Cys Asp Lys Thr His Thr Cys Pro Pro Cys
            100                 105                 110


Pro Ala Pro Glu Leu Leu Gly Gly Asp Ser Val Phe Leu Phe Pro Pro
            115                 120                 125


Lys Pro Lys Asp Val Leu Met Ile Ser Arg Thr Pro Glu Val Thr Cys
        130                 135                 140


Val Val Ile Asp Val Ser His Glu Asp Pro Glu Val Lys Phe Asn Trp
145                 150                 155                 160


Tyr Val Asp Gly Val Glu Val His Asn Ala Lys Thr Lys Pro Arg Glu
                165                 170                 175


Glu Gln Tyr Asn Ser Thr Tyr Arg Val Val Ser Val Leu Pro Val Leu
                180                 185                 190

```
His Arg Asp Trp Leu Asn Gly Lys Glu Tyr Lys Cys Lys Val Ser Asn
        195                 200                 205

Lys Ala Leu Pro Lys Pro Ile Glu Lys Thr Ile Ser Lys Ala Lys Gly
        210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
        290                 295                 300

Val Phe Ser Cys Ser Val Leu His Glu Ala Leu His Ala His Tyr Thr
305                 310                 315                 320

Arg Lys Glu Leu Ser Leu Ser Pro
                325
```

```
<210>   52
<211>   328
<212>   PRT
<213>   Artificial Sequence

<220>
<223>   IgG heavy chain constant region

<400>   52
```

```
Ala Ser Thr Lys Gly Pro Ser Val Phe Pro Leu Ala Pro Ser Ser Lys
1                   5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Ser Leu Gly Thr Gln Thr
```

|  | 65 | | | | 70 | | | | 75 | | | | 80 |
|---|---|---|---|---|---|---|---|---|---|---|---|---|---|

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Asp Ser Val Phe Leu Phe Pro Pro
115 120 125

Lys Pro Lys Asp Val Leu Met Ile Ser Arg Thr Pro Glu Val Thr Cys
130 135 140

Val Val Val Asp Val Ala His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Pro Val Leu
180 185 190

His Arg Asp Trp Leu Asn Gly Lys Glu Tyr Lys Cys Lys Val Ser Asn
195 200 205

Lys Ala Leu Pro Lys Pro Ile Glu Lys Thr Ile Ser Lys Ala Lys Gly
210 215 220

Gln Arg Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Leu His Glu Ala Leu His Ala His Tyr Thr
305 310 315 320

Arg Lys Glu Leu Ser Leu Ser Pro
                325

<210> 53
<211> 328
<212> PRT
<213> Artificial Sequence

<220>
<223> IgG heavy chain constant region

<400> 53

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Tyr Leu Gly Gly Asp Ser Val Phe Leu Phe Pro Pro
            115                 120                 125

Lys Pro Lys Asp Val Leu Met Ile Ser Arg Thr Pro Glu Val Thr Cys
        130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
            180                 185                 190

His Arg Asp Trp Leu Asn Gly Lys Glu Tyr Lys Cys Lys Val Ser Asn

                    195                    200                    205

Lys Ala Leu Pro Lys Pro Ile Glu Lys Thr Ile Ser Lys Ala Lys Gly
    210                215                220

Gln Arg Arg Glu Pro Gln Val Tyr Thr Leu Pro Pro Ser Arg Glu Glu
225                230                235                240

Met Thr Lys Asn Gln Val Ser Leu Thr Cys Leu Val Lys Gly Phe Tyr
                245                250                255

Pro Ser Asp Ile Ala Val Glu Trp Glu Ser Asn Gly Gln Pro Glu Asn
                260                265                270

Asn Tyr Lys Thr Thr Pro Pro Val Leu Asp Ser Asp Gly Ser Phe Phe
        275                280                285

Leu Tyr Ser Lys Leu Thr Val Asp Lys Ser Arg Trp Gln Gln Gly Asn
    290                295                300

Val Phe Ser Cys Ser Val Leu His Glu Ala Leu His Ala His Tyr Thr
305                310                315                320

Arg Lys Glu Leu Ser Leu Ser Pro
                325

<210> 54
<211> 328
<212> PRT
<213> Artificial Sequence

<220>
<223> IgG heavy chain constant region

<400> 54

Ala Ser Thr Lys Gly Pro Ser Val Phe Pro Leu Ala Pro Ser Ser Lys
1                5                10                15

Ser Thr Ser Gly Gly Thr Ala Ala Leu Gly Cys Leu Val Lys Asp Tyr
                20                25                30

Phe Pro Glu Pro Val Thr Val Ser Trp Asn Ser Gly Ala Leu Thr Ser
                35                40                45

Gly Val His Thr Phe Pro Ala Val Leu Gln Ser Ser Gly Leu Tyr Ser
        50                55                60

Leu Ser Ser Val Val Thr Val Pro Ser Ser Ser Leu Gly Thr Gln Thr
65                70                75                80

```
Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
                100                 105                 110

Pro Ala Pro Glu Tyr Leu Gly Gly Asp Ser Val Phe Leu Phe Pro Pro
                115                 120                 125

Lys Pro Lys Asp Val Leu Met Ile Ser Arg Thr Pro Glu Val Thr Cys
                130                 135                 140

Val Val Val Asp Val Ala His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
                180                 185                 190

His Arg Asp Trp Leu Asn Gly Lys Glu Tyr Lys Cys Lys Val Ser Asn
                195                 200                 205

Lys Ala Leu Pro Lys Pro Ile Glu Lys Thr Ile Ser Lys Ala Lys Gly
                210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
                260                 265                 270

Asn Tyr Lys Thr Thr Pro Pro Val Leu Asp Ser Asp Gly Ser Phe Phe
                275                 280                 285

Leu Tyr Ser Lys Leu Thr Val Asp Lys Ser Arg Trp Gln Gln Gly Asn
                290                 295                 300

Val Phe Ser Cys Ser Val Leu His Glu Ala Leu His Ala His Tyr Thr
305                 310                 315                 320

Arg Lys Glu Leu Ser Leu Ser Pro
```

325

```
<210>  55
<211>  328
<212>  PRT
<213>  Artificial Sequence

<220>
<223>  IgG heavy chain constant region

<400>  55
```

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
            35                  40                  45

Gly Val His Thr Phe Pro Ala Val Leu Gln Ser Ser Gly Leu Tyr Ser
            50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Asp Ser Val Phe Leu Phe Pro Pro
            115                 120                 125

Lys Pro Lys Asp Val Leu Met Ile Ser Arg Thr Pro Glu Val Thr Cys
            130                 135                 140

Val Val Ile Asp Val Ala His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
                180                 185                 190

His Arg Asp Trp Leu Asn Gly Lys Glu Tyr Lys Cys Lys Val Ser Asn
            195                 200                 205

```
Lys Ala Leu Pro Lys Pro Ile Glu Lys Thr Ile Ser Lys Ala Lys Gly
    210                 215                 220

Gln Arg Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Leu His Glu Ala Leu His Ala His Tyr Thr
305                 310                 315                 320

Arg Lys Glu Leu Ser Leu Ser Pro
                325
```

```
<210>  56
<211>  328
<212>  PRT
<213>  Artificial Sequence

<220>
<223>  IgG heavy chain constant region

<400>  56
```

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80
```

92

```
        Tyr Ile Cys Asn Val Asn His Lys Pro Ser Asn Thr Lys Val Asp Lys
                        85              90                  95


        Lys Val Glu Pro Lys Ser Cys Asp Lys Thr His Thr Cys Pro Pro Cys
                    100             105                 110


        Pro Ala Pro Glu Tyr Leu Gly Gly Asp Ser Val Phe Leu Phe Pro Pro
                    115             120                 125


        Lys Pro Lys Asp Val Leu Met Ile Ser Arg Thr Pro Glu Val Thr Cys
                130             135                 140


        Val Val Ile Asp Val Ser His Glu Asp Pro Glu Val Lys Phe Asn Trp
        145             150                 155                 160


        Tyr Val Asp Gly Val Glu Val His Asn Ala Lys Thr Lys Pro Arg Glu
                        165             170                 175


        Glu Gln Tyr Asn Ser Thr Tyr Arg Val Val Ser Val Leu Pro Val Leu
                    180             185                 190


        His Arg Asp Trp Leu Asn Gly Lys Glu Tyr Lys Cys Lys Val Ser Asn
                    195             200                 205


        Lys Ala Leu Pro Lys Pro Ile Glu Lys Thr Ile Ser Lys Ala Lys Gly
                    210             215                 220


        Gln Arg Arg Glu Pro Gln Val Tyr Thr Leu Pro Pro Ser Arg Glu Glu
        225             230                 235                 240


        Met Thr Lys Asn Gln Val Ser Leu Thr Cys Leu Val Lys Gly Phe Tyr
                        245             250                 255


        Pro Ser Asp Ile Ala Val Glu Trp Glu Ser Asn Gly Gln Pro Glu Asn
                        260             265                 270


        Asn Tyr Lys Thr Thr Pro Pro Val Leu Asp Ser Asp Gly Ser Phe Phe
                    275             280                 285


        Leu Tyr Ser Lys Leu Thr Val Asp Lys Ser Arg Trp Gln Gln Gly Asn
                290             295                 300


        Val Phe Ser Cys Ser Val Leu His Glu Ala Leu His Ala His Tyr Thr
        305             310                 315                 320


        Arg Lys Glu Leu Ser Leu Ser Pro
                        325
```

```
<210>  57
<211>  328
<212>  PRT
<213>  Artificial Sequence

<220>
<223>  IgG heavy chain constant region

<400>  57
```

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
            35                  40                  45

Gly Val His Thr Phe Pro Ala Val Leu Gln Ser Ser Gly Leu Tyr Ser
            50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Tyr Leu Gly Gly Asp Ser Val Phe Leu Phe Pro Pro
            115                 120                 125

Lys Pro Lys Asp Val Leu Met Ile Ser Arg Thr Pro Glu Val Thr Cys
            130                 135                 140

Val Val Ile Asp Val Ala His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Pro Val Leu
                180                 185                 190

His Arg Asp Trp Leu Asn Gly Lys Glu Tyr Lys Cys Lys Val Ser Asn
            195                 200                 205
```

94

```
Lys Ala Leu Pro Lys Pro Ile Glu Lys Thr Ile Ser Lys Ala Lys Gly
    210             215             220

Gln Arg Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
                245             250             255

Pro Ser Asp Ile Ala Val Glu Trp Glu Ser Asn Gly Gln Pro Glu Asn
                260             265             270

Asn Tyr Lys Thr Thr Pro Pro Val Leu Asp Ser Asp Gly Ser Phe Phe
            275             280             285

Leu Tyr Ser Lys Leu Thr Val Asp Lys Ser Arg Trp Gln Gln Gly Asn
    290             295             300

Val Phe Ser Cys Ser Val Leu His Glu Ala Leu His Ala His Tyr Thr
305             310             315             320

Arg Lys Glu Leu Ser Leu Ser Pro
                325
```

```
<210>   58
<211>   330
<212>   PRT
<213>   Homo sapiens

<400>   58
```

```
Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
    50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90              95
```

Lys Val Glu Pro Lys Ser Cys Asp Lys Thr His Thr Cys Pro Pro Cys
                100                 105                 110

Pro Ala Pro Glu Leu Leu Gly Gly Pro Ser Val Phe Leu Phe Pro Pro
                115                 120                 125

Lys Pro Lys Asp Thr Leu Met Ile Ser Arg Thr Pro Glu Val Thr Cys
                130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
                180                 185                 190

His Gln Asp Trp Leu Asn Gly Lys Glu Tyr Lys Cys Lys Val Ser Asn
                195                 200                 205

Lys Ala Leu Pro Ala Pro Ile Glu Lys Thr Ile Ser Lys Ala Lys Gly
                210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
                260                 265                 270

Asn Tyr Lys Thr Thr Pro Pro Val Leu Asp Ser Asp Gly Ser Phe Phe
                275                 280                 285

Leu Tyr Ser Lys Leu Thr Val Asp Lys Ser Arg Trp Gln Gln Gly Asn
                290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>   59
<211>   326

<212> PRT
<213> Homo sapiens

<400> 59

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
        130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
        210                 215                 220

Pro Gln Val Tyr Thr Leu Pro Pro Ser Arg Glu Glu Met Thr Lys Asn
225                 230                 235                 240

```
Gln Val Ser Leu Thr Cys Leu Val Lys Gly Phe Tyr Pro Ser Asp Ile
              245                 250                 255

Ala Val Glu Trp Glu Ser Asn Gly Gln Pro Glu Asn Asn Tyr Lys Thr
              260                 265                 270

Thr Pro Pro Met Leu Asp Ser Asp Gly Ser Phe Phe Leu Tyr Ser Lys
              275                 280                 285

Leu Thr Val Asp Lys Ser Arg Trp Gln Gln Gly Asn Val Phe Ser Cys
              290                 295                 300

Ser Val Met His Glu Ala Leu His Asn His Tyr Thr Gln Lys Ser Leu
305                 310                 315                 320

Ser Leu Ser Pro Gly Lys
                  325


<210>  60
<211>  377
<212>  PRT
<213>  Homo sapiens

<400>  60

Ala Ser Thr Lys Gly Pro Ser Val Phe Pro Leu Ala Pro Cys Ser Arg
1                   5                   10                  15

Ser Thr Ser Gly Gly Thr Ala Ala Leu Gly Cys Leu Val Lys Asp Tyr
              20                  25                  30

Phe Pro Glu Pro Val Thr Val Ser Trp Asn Ser Gly Ala Leu Thr Ser
              35                  40                  45

Gly Val His Thr Phe Pro Ala Val Leu Gln Ser Ser Gly Leu Tyr Ser
              50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                  85                  90                  95

Arg Val Glu Leu Lys Thr Pro Leu Gly Asp Thr Thr His Thr Cys Pro
              100                 105                 110

Arg Cys Pro Glu Pro Lys Ser Cys Asp Thr Pro Pro Pro Cys Pro Arg
              115                 120                 125
```

Cys Pro Glu Pro Lys Ser Cys Asp Thr Pro Pro Pro Cys Pro Arg Cys
    130                 135             140

Pro Glu Pro Lys Ser Cys Asp Thr Pro Pro Pro Cys Pro Arg Cys Pro
145             150             155                 160

Ala Pro Glu Leu Leu Gly Gly Pro Ser Val Phe Leu Phe Pro Pro Lys
            165             170             175

Pro Lys Asp Thr Leu Met Ile Ser Arg Thr Pro Glu Val Thr Cys Val
        180             185             190

Val Val Asp Val Ser His Glu Asp Pro Glu Val Gln Phe Lys Trp Tyr
    195             200             205

Val Asp Gly Val Glu Val His Asn Ala Lys Thr Lys Pro Arg Glu Glu
    210             215             220

Gln Tyr Asn Ser Thr Phe Arg Val Val Ser Val Leu Thr Val Leu His
225             230             235             240

Gln Asp Trp Leu Asn Gly Lys Glu Tyr Lys Cys Lys Val Ser Asn Lys
            245             250             255

Ala Leu Pro Ala Pro Ile Glu Lys Thr Ile Ser Lys Thr Lys Gly Gln
        260             265             270

Pro Arg Glu Pro Gln Val Tyr Thr Leu Pro Pro Ser Arg Glu Glu Met
    275             280             285

Thr Lys Asn Gln Val Ser Leu Thr Cys Leu Val Lys Gly Phe Tyr Pro
    290             295             300

Ser Asp Ile Ala Val Glu Trp Glu Ser Ser Gly Gln Pro Glu Asn Asn
305             310             315             320

Tyr Asn Thr Thr Pro Pro Met Leu Asp Ser Asp Gly Ser Phe Phe Leu
            325             330             335

Tyr Ser Lys Leu Thr Val Asp Lys Ser Arg Trp Gln Gln Gly Asn Ile
        340             345             350

Phe Ser Cys Ser Val Met His Glu Ala Leu His Asn Arg Phe Thr Gln
    355             360             365

Lys Ser Leu Ser Leu Ser Pro Gly Lys

370                          375

<210>   61
<211>   327
<212>   PRT
<213>   Homo sapiens

<400>   61

Ala Ser Thr Lys Gly Pro Ser Val Phe Pro Leu Ala Pro Cys Ser Arg
1                 5                 10                15

Ser Thr Ser Glu Ser Thr Ala Ala Leu Gly Cys Leu Val Lys Asp Tyr
              20                25                30

Phe Pro Glu Pro Val Thr Val Ser Trp Asn Ser Gly Ala Leu Thr Ser
          35                40                45

Gly Val His Thr Phe Pro Ala Val Leu Gln Ser Ser Gly Leu Tyr Ser
      50                55                60

Leu Ser Ser Val Val Thr Val Pro Ser Ser Ser Leu Gly Thr Lys Thr
65                70                75                80

Tyr Thr Cys Asn Val Asp His Lys Pro Ser Asn Thr Lys Val Asp Lys
              85                90                95

Arg Val Glu Ser Lys Tyr Gly Pro Pro Cys Pro Ser Cys Pro Ala Pro
          100               105               110

Glu Phe Leu Gly Gly Pro Ser Val Phe Leu Phe Pro Pro Lys Pro Lys
      115               120               125

Asp Thr Leu Met Ile Ser Arg Thr Pro Glu Val Thr Cys Val Val Val
      130               135               140

Asp Val Ser Gln Glu Asp Pro Glu Val Gln Phe Asn Trp Tyr Val Asp
145               150               155               160

Gly Val Glu Val His Asn Ala Lys Thr Lys Pro Arg Glu Glu Gln Phe
          165               170               175

Asn Ser Thr Tyr Arg Val Val Ser Val Leu Thr Val Leu His Gln Asp
          180               185               190

Trp Leu Asn Gly Lys Glu Tyr Lys Cys Lys Val Ser Asn Lys Gly Leu
      195               200               205

Pro Ser Ser Ile Glu Lys Thr Ile Ser Lys Ala Lys Gly Gln Pro Arg

```
                210                    215                       220

        Glu Pro Gln Val Tyr Thr Leu Pro Pro Ser Gln Glu Glu Met Thr Lys
        225                 230                 235                 240


        Asn Gln Val Ser Leu Thr Cys Leu Val Lys Gly Phe Tyr Pro Ser Asp
                        245                 250                 255


        Ile Ala Val Glu Trp Glu Ser Asn Gly Gln Pro Glu Asn Asn Tyr Lys
                        260                 265                 270


        Thr Thr Pro Pro Val Leu Asp Ser Asp Gly Ser Phe Phe Leu Tyr Ser
                275                 280                 285


        Arg Leu Thr Val Asp Lys Ser Arg Trp Gln Glu Gly Asn Val Phe Ser
                290                 295                 300


        Cys Ser Val Met His Glu Ala Leu His Asn His Tyr Thr Gln Lys Ser
        305                 310                 315                 320


        Leu Ser Leu Ser Leu Gly Lys
                        325



        <210>   62
        <211>   328
        <212>   PRT
        <213>   Artificial sequence

        <220>
        <223>   An artificially synthesized sequence

        <400>   62

        Ala Ser Thr Lys Gly Pro Ser Val Phe Pro Leu Ala Pro Ser Ser Lys
        1                   5                   10                  15


        Ser Thr Ser Gly Gly Thr Ala Ala Leu Gly Cys Leu Val Lys Asp Tyr
                        20                  25                  30


        Phe Pro Glu Pro Val Thr Val Ser Trp Asn Ser Gly Ala Leu Thr Ser
                35                  40                  45


        Gly Val His Thr Phe Pro Ala Val Leu Gln Ser Ser Gly Leu Tyr Ser
                50                  55                  60


        Leu Ser Ser Val Val Thr Val Pro Ser Ser Ser Leu Gly Thr Gln Thr
        65                  70                  75                  80


        Tyr Ile Cys Asn Val Asn His Lys Pro Ser Asn Thr Lys Val Asp Lys
                        85                  90                  95
```

```
Lys Val Glu Pro Lys Ser Cys Asp Lys Thr His Thr Cys Pro Pro Cys
            100             105             110

Pro Ala Pro Glu Leu Leu Gly Gly Pro Ser Val Phe Leu Phe Pro Pro
            115             120             125

Lys Pro Lys Asp Thr Leu Met Ile Ser Arg Thr Pro Glu Val Thr Cys
        130             135             140

Val Val Val Asp Val Ser His Glu Asp Pro Glu Val Lys Phe Asn Trp
145             150             155             160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Thr Val Leu
            180             185             190

His Gln Asp Trp Leu Asn Gly Lys Glu Tyr Lys Cys Lys Val Ser Asn
            195             200             205

Lys Ala Leu Pro Ala Pro Ile Glu Lys Thr Ile Ser Lys Ala Lys Gly
        210             215             220

Gln Pro Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Gln Gly Asn
        290             295             300

Val Phe Ser Cys Ser Val Met His Glu Ala Leu His Asn His Tyr Thr
305             310             315             320

Gln Lys Ser Leu Ser Leu Ser Pro
                325
```

**Description**

[Technical Field]

The present invention relates to methods of purifying an antibody and to methods for purifying an Fc region-modified antibody comprising specific amino acid mutations.

[Background Art]

With the development of gene recombination technology, various protein preparations can now be supplied in stable amounts, and various therapeutic antibodies are being developed.

When an antibody is produced using mammalian cells as host by gene recombination technology, it is subjected to Protein A or Protein G affinity column chromatography by utilizing the property of Protein A or Protein G to bind to the Fc chain of IgG, after which, purification is carried out by various chromatographies. In particular, purification of an antibody by Protein A affinity column chromatography is the process most commonly used in the production of therapeutic antibody to recover the antibody from the culture medium.

For example, in JP-A (Kohyo) H05-504579 (PTL 1), an antibody-containing aqueous medium obtained from a mammalian cell culture was applied to Protein A or Protein G column chromatography to allow adsorption of the antibody onto the column, then the antibody was eluted with an acidic solution (citric acid with a concentration of about 0.1 M, pH 3.0-3.5), and the obtained acidic eluate was sequentially applied to ion exchange column chromatography and to size exclusion column chromatography for purification.

On the other hand, for the purpose of improving blood retention or *in vivo* kinetics, amino acid substitution techniques for regulating the isoelectric point (pI) of an antibody, specifically, techniques for regulating the pI of an antibody by modifying amino acid residues exposed on the surface of an antibody, are known (WO 07/114319 (PTL 2), WO 2017/104783 (PTL 3)). PTL 2 discloses that modification of amino acid residues of an antibody to regulate the pI is expected to improve plasma retention and half-life of the antibody, and that this leads to a reduction of the dose and the extension of the administration interval of the antibody as a medicament. Further, PTL 3 discloses that by introducing the amino acid substitutions Q311R and P343R into the CH2 region and CH3 region of an antibody to modify the antibody to increase the pI, antigen elimination from plasma can be enhanced when the antibody is administered *in vivo*.

Protein A used for antibody purification is a protein present in the cell wall of *Staphylococcus aureus* and binds to immunoglobulins, especially to the Fc region of IgG. In general, the Protein A protein derived from *Staphylococcus* has a repeated structure including five immunoglobulin-binding domains having homology to each other, called the E-domain, D-domain, A-domain, B-domain, and C-domain, and each binding domain can bind singly to an immunoglobulin. Along with natural Protein A, recombinant proteins consisting only of immunoglobulin-binding domain(s) with partially-modified amino acids are also used as affinity ligands for affinity chromatography. For example, Protein A columns with improved antibody purification efficiency have been developed by substituting any one or more originally present lysines at positions 4, 7, and 35 of a C-domain variant or Z-domain with an amino acid other than lysine, where the C-domain variant was prepared by substituting the glycine at position 29 of the amino acid sequence of the C-domain of *Staphylococcus* Protein A with alanine, and the Z-domain was prepared by substituting the glycine at position 29 of the amino acid sequence of the B-domain of *Staphylococcus* Protein A with alanine (PTLs 4 and 5).

[Citation List]

[Patent Literature]

[PTL 1] Japanese Patent Application *Kohyo* Publication No. (JP-A) H05-504579 (unexamined Japanese national phase publication corresponding to a non-Japanese international publication)
[PTL 2] WO 2007/11431
[PTL 3] WO 2017/104783
[PTL 4] Japanese Patent Application *Kokai* Publication No. (JP-A) 2007-252368 (unexamined, published Japanese Patent Application)
[PTL 5] WO 2015/034000

[Summary of Invention]

[Technical Problem]

Protein A has been conventionally utilized as a ligand for antibody purification. However, evaluation of purification methods suitable for antibodies with amino acid modifications for modifying pI (hereinafter referred to as pI-modified antibodies) and issues in the purification process have not been investigated in detail so far. Therefore, for example, it was not known that there are antibodies that cannot be purified by commonly-used Protein A columns.

The present inventors discovered that there are pI-modified antibodies that cannot be efficiently purified by the commonly-used Protein A columns. An efficient purification method suitable for such antibodies is thus needed. In other words, it is an objective of the present invention to provide a highly efficient and economical purification method which enables production of an antibody on an industrial scale even when the antibody is a pI-modified antibody that cannot be efficiently purified by a common Protein A column.

[Solution to Problem]

As a result of diligent research to achieve the above objective, the present inventors discovered that the use of a resin comprising a specific modified Protein A ligand enables efficient purification of even pI-modified antibodies that cannot be efficiently purified with a commonly-used Protein A column.

More specifically, the present invention provides the following [1] to [20]:

[1] a method of purifying an IgG antibody comprising the amino acid residue substitutions Q311R and P343R from a composition containing the antibody, wherein the method comprises the steps of:

(a) preparing an affinity column containing a carrier onto which a Protein A-modified ligand is immobilized, wherein the Protein A-modified ligand comprises: a modified immunoglobulin-binding domain comprising a modification for substitution of any one or more originally present lysine residues at positions 4, 7, and 35 of the C-domain variant of *Staphylococcus* Protein A of SEQ ID NO: 1 or Z-domain of *Staphylococcus* Protein A of SEQ ID NO: 2 with amino acid residues other than lysine; or a multimer of these modified immunoglobulin-binding domains;
(b) loading the composition containing the IgG antibody onto the affinity column of step (a); and
(c) eluting and recovering the IgG antibody from the affinity column of step (b);

[2] the method of [1], wherein the multimer of the modified immunoglobulin-binding domains is a dimer to decamer, and wherein arranged at the first or second from the N-terminal or C-terminal side in the multimer is an immunoglobulin-binding domain in which at least one of the originally present amino acid residues at positions 40, 43, 46, 53, 54, and 56 of the C-domain variant (SEQ ID NO: 1) or Z-domain (SEQ ID NO: 2) has been substituted with a lysine residue;
[3] the method of [1] or [2], wherein the substitution is a modification for substitution of any one or more originally present lysine residues at positions 4, 7, and 35 of the C-domain variant or Z-domain of Protein A with any one of amino acid residues selected from the group consisting of an alanine residue (A), a glutamine residue (Q), an asparagine residue (D), a valine residue (V), a serine residue (S), a threonine residue (T), a histidine residue (H), a tyrosine residue (Y), an arginine residue (R), a glutamic acid residue (E), a phenylalanine residue (F), a leucine residue (L), an isoleucine residue (I), and a proline residue (P);
[4] the method of any one of [1] to [3], wherein the modified immunoglobulin-binding domain is (i) a modified immunoglobulin-binding domain comprising the amino acid sequence of SEQ ID NO: 3 or 5; or (ii) a modified immunoglobulin-binding domain comprising an amino acid sequence in which one to several amino acid residues have been substituted, deleted, added, and/or inserted to the amino acid sequence of SEQ ID NO: 3 or 5 at amino acid residues other than those at positions 4, 7, and 35;
[5] the method of any one of [1] to [4], wherein the modified immunoglobulin-binding domain has an ability to bind to an IgG antibody comprising the amino acid residue substitutions Q311R and P343R;
[6] the method of any one of [1] to [3], wherein the Protein A-modified ligand is a modified ligand comprising a modified immunoglobulin-binding domain that consists of at least one amino acid sequence selected from the group consisting of SEQ ID NOs: 3, 4, and 5;
[7] the method of any one of [1] to [6], wherein the Protein A-modified ligand is immobilized onto the carrier by any one means selected from the group consisting of (1) to (5) below:

(1) a method of immobilization onto the carrier through a modified immunoglobulin-binding domain in which 1 to 6 of the amino acid residues at positions 40, 43, 46, 53, 54, and 56 in the C-domain or Z-domain of Protein A are additionally substituted with a lysine residue;

(2) a method of immobilization onto the carrier through a disulfide bond or a thioether bond by introducing cysteine into the C-terminus of Protein A;

(3) a method of immobilization onto an amino group-containing immobilization carrier by cyanation of a thiol group;

(4) a method of immobilizing a multimer of modified immunoglobulin-binding domains having a cysteine residue onto an amino group-containing carrier using 4-(N-maleimidomethyl)cyclohexane-1-carboxylate (SMCC) as a cross-linking agent; and

(5) a method of immobilization onto the carrier through a plurality of lysine residues added to the C-terminus of a modified immunoglobulin-binding domain in which the lysine residues at positions 42, 49, 50, and 58 of the C-domain variant of Protein A are substituted with amino acids other than lysine, or to a modified immunoglobulin-binding domain in which the lysine residues at positions 49, 50, and 58 of the Z-domain are substituted with amino acids other than lysine;

[8] the method of any one of [1] to [7], wherein the IgG antibody is an IgG antibody additionally comprising one or more amino acid residue substitutions selected from among M428L, N434A, Y436T, Q438R, and S440E in the CH3 region of the IgG antibody;

[9] the method of any one of [1] to [8], wherein the IgG antibody is one in which the CH3 region of the IgG antibody comprises an amino acid sequence selected from the group consisting of SEQ ID NOs: 6 to 11;

[10] the method of any one of [1] to [9], wherein the IgG antibody is one in which the heavy chain constant region of the IgG antibody comprises an amino acid sequence selected from the group consisting of SEQ ID NOs: 12 to 57;

[11] the method of any one of [1] to [10], wherein the pI value of the IgG antibody is 4.0 to 10.0;

[12] the method of any one of [1] to [11], wherein the amount of the Protein A-modified ligand bound to the Fc region of the IgG antibody is 5 times or more compared to the binding ability to unmodified Protein A;

[13] the method of any one of [1] to [12], wherein the method additionally comprises the step of washing the affinity column with a washing solution before step (c);

[14] the method of [13], wherein the washing solution is a combination of a buffer and a salt, and contains, as the buffer, at least one selected from the group consisting of phosphoric acid, acetic acid, citric acid, glycine, and tris hydroxymethyl aminomethane, and as the salt, at least one selected from the group consisting of arginine, sodium chloride, and sodium sulfate;

[15] the method of any one of [1] to [14], wherein the method additionally comprises, after step (c), the step of purifying the IgG antibody by at least one chromatography selected from the group consisting of cation exchange chromatography, anion exchange chromatography, hydrophobic interaction chromatography, multimode chromatography, and hydroxyapatite chromatography;

[16] the method of any one of [1] to [15], wherein step (c) comprises the step of eluting the IgG antibody from the affinity column with an eluting solution containing at least one selected from the group consisting of hydrochloric acid, acetic acid, citric acid, arginine, glycine, and phosphoric acid;

[17] the method of any one of [1] to [16], wherein the antibody is a humanized antibody or a human antibody;

[18] the method of any one of [1] to [17], wherein the antibody is an anti-myostatin antibody, an anti-IL-6 receptor antibody, an anti-IL-6 antibody, an anti-IL-8 antibody, or an anti-IL-31 receptor antibody;

[19] use of an affinity column containing a carrier onto which a Protein A-modified ligand has been immobilized, in the purification of an IgG antibody comprising the amino acid residue substitutions Q311R and P343R, wherein the protein A-modified ligand comprises either or both of an amino acid-substituted C-domain variant and Z-domain of Protein A, wherein the substitution is a substitution which alters any one or more originally present lysine residues at positions 4, 7, and 35 of the C-domain variant or Z-domain to amino acid residues other than lysine, and wherein the protein A-modified ligand is a modified ligand having an ability to bind to the IgG antibody;

[20] a method of producing an IgG antibody having the amino acid residue substitutions Q311R and P343R, wherein the method comprises the following steps of:

(i) providing a composition containing an IgG antibody comprising the amino acid residue substitutions Q311R and P343R;

(ii) preparing an affinity column containing a carrier onto which a Protein A-modified ligand is immobilized, wherein the Protein A-modified ligand comprises a modified immunoglobulin-binding domain comprising a modification for substitution of any one or more originally present lysine residues at positions 4, 7, and 35 of the C-domain variant of *Staphylococcus* Protein A of SEQ ID NO: 1 or the Z-domain of *Staphylococcus* Protein A of SEQ ID NO: 2 with amino acid residues other than lysine; or a multimer of these modified immunoglobulin-binding domains;

(iii) loading the composition containing the IgG antibody onto the affinity column of step (ii); and
(iv) eluting and recovering the IgG antibody from the affinity column loaded with the composition containing the IgG antibody in step (iii).

Alternatively, the present invention provides:

[1'] a method of purifying an IgG antibody comprising the amino acid residue substitutions Q311R and P343R from a composition containing the antibody, wherein the method comprises the steps of:

(a) preparing an affinity column containing a carrier onto which a Protein A-modified ligand is immobilized;
(b) loading the composition containing the IgG antibody onto the affinity column of step (a); and
(c) eluting and recovering the IgG antibody from the affinity column of step (b),

wherein the Protein A-modified ligand contains either or both of an amino acid-substituted C-domain variant and Z-domain of Protein A, wherein the substitution comprises a substitution which alters any one or more originally present lysine residues at positions 4, 7, and 35 of the C-domain variant or Z-domain to amino acid residues other than lysine, and is a Protein A-modified ligand having an activity to bind to the IgG antibody.

Advantageous Effect of Invention

By the present invention, even pI-modified antibodies that cannot be successfully purified by a common Protein A column can be purified easily and efficiently.

[Brief Description of Drawings]

Fig. 1 shows the results of measuring the dynamic binding capacity (DBC) of an antibody that does not contain a modification in the Fc region in each Protein A-immobilized resin. In the figure, the vertical axis shows DBC (g/L resin), and the horizontal axis shows residence time (minutes).
Fig. 2-1 shows the result (real-time binding curve) of evaluating the binding affinity between the ligand (structure represented by Formula (1')) for AF-rProtein A HC-650F and an antibody, using the BLItz (registered trademark) evaluation system (ForteBio).
Fig. 2-2 shows the result (real-time binding curve) of evaluating the binding affinity between the ligand for MabSelect SuRe and an antibody, using the BLItz (registered trademark) evaluation system (ForteBio).

[Description of Embodiments]

Hereinbelow, the present invention will be described in detail.
The present invention relates to methods of purifying a composition containing a pI-modified antibody having an increased isoelectric point (pI). Specifically, the present invention relates to methods of purifying an IgG antibody comprising the amino acid residue substitutions Q311R and P343R from a composition containing the antibody, wherein the method comprises the following steps of:

(a) preparing an affinity column containing a carrier onto which a Protein A-modified ligand is immobilized, wherein the Protein A-modified ligand comprises: a modified immunoglobulin-binding domain containing a modification for substitution of any one or more originally present lysine residues at positions 4, 7, and 35 of the C-domain variant of *Staphylococcus* Protein A of SEQ ID NO: 1 or the Z-domain of *Staphylococcus* Protein A of SEQ ID NO: 2 with amino acid residues other than lysine; or a multimer of these modified immunoglobulin-binding domains;
(b) loading the composition containing the IgG antibody onto the affinity column of step (a); and
(c) eluting and recovering the IgG antibody loaded in step (b).

The IgG antibodies comprising the amino acid residue substitutions Q311R and P343R with regard to the CH2 and CH3 regions in the present invention are antibodies in which both glutamine (Q) at position 311 and proline (P) at position 343 of the CH2 and CH3 regions (according to EU numbering) in the parent Fc region have been modified to arginine (R). In general, the CH2 region corresponds to the amino acids at positions 231 to 340, and the CH3 region corresponds to the amino acids at positions 341 to 447 (according to EU numbering) within the hinge region. The "parent Fc region" in the present application refers to an Fc region before the introduction of the amino acid modifications described in the present specification. Preferred examples of the parent Fc region include Fc regions derived from natural antibodies. Antibodies can be derived from humans or monkeys (e.g., cynomolgus monkeys, rhesus monkeys, marmosets, chimpanzees, or baboons). Natural antibodies may comprise naturally occurring mutations. Multiple allotype sequences of IgG due to genetic polymorphisms are described in "Sequences of Proteins of Immunological Interest", NIH Publication No. 91-3242, all of which can be used in the present invention. In particular, for human IgG1, the amino acid sequence at positions 356-358 (EU numbering) can be either DEL or EEM. Preferred examples of the parent Fc region include an Fc region derived from a heavy chain constant region of human IgG1 (SEQ ID NO: 58), human IgG2 (SEQ ID NO: 59), human IgG3 (SEQ ID NO: 60) and human IgG4 (SEQ ID NO: 61). Another preferred example of the parent Fc region is an Fc region derived from the heavy chain constant region SG1 (SEQ ID NO: 62). Further, the parent Fc region may be an Fc region prepared by adding amino acid modifications other than the amino acid modifications described in the present specification to an Fc region derived from the natural antibody.

With respect to the antibody in the present invention, amino acid modifications carried out for other purposes may be combined with the antibodies used in the present invention. For example, amino acid substitutions that enhance FcRn-binding activity (Hinton et al., J. Immunol. 176(1): 346-356 (2006); Dall'Acqua et al., J. Biol. Chem. 281(33): 23514-23524 (2006); Petkova et al., Intl. Immunol. 18(12): 1759-1769 (2006); Zalevsky et al., Nat. Biotechnol. 28(2): 157-159 (2010); WO 2006/019447; WO 2006/053301; and WO 2009/086320), and amino acid substitutions for improving antibody heterogeneity or stability (WO 2009/041613) may be added. Alternatively, amino acid modifications applied to polypeptides having properties that promote antigen clearance as described in WO 2011/122011, WO 2012/132067, WO 2013/046704, or WO 2013/180201, polypeptides having specific binding properties to target tissues as described in WO 2013/180200, or polypeptides having the property of repeatedly binding to multiple antigen molecules as described in WO 2009/125825, WO 2012/073992, or WO 2013/047752 may be combined with the antibodies used in the present invention. The amino acid modifications disclosed in EP1752471 and EP1772465 may be combined with the antibodies used in the present invention for the purpose of imparting binding ability to other antigens. Amino acid modifications that lower the pI of the constant region (WO 2012/016227) may be combined with the antibodies used in the present invention for the purpose of increasing plasma retention. Amino acid modifications that increase the pI of the constant region (WO 2014/145159) may be combined for the purpose of promoting uptake into cells. Amino acid modifications that increase the pI of the constant region (Japanese Patent Application Nos. 2015-021371 and 2015-185254) may be combined for the purpose of promoting the elimination of a target molecule from plasma.

In the present invention, amino acid modification means any substitution, deletion, addition, insertion, and modifications, or combinations thereof. In the present invention, an amino acid modification can be paraphrased as an amino acid mutation.

The antibodies used in the present invention are more preferably IgG antibodies which further comprise one or more amino acid residue substitutions selected from M428L, N434A, Y436T, Q438R, and S440E in the CH3 region, and even more preferably, they are IgG antibodies which comprise in the CH3 region two or more amino acid residue substitutions selected from M428L, N434A, Y436T, Q438R, and S440E. Further preferably, they include IgG antibodies where the CH3 region comprises an amino acid sequence selected from the group consisting of SEQ ID NOs: 6 to 11, and IgG antibodies where the heavy chain constant region comprises an amino acid sequence selected from the group consisting of SEQ ID NOs: 12 to 57.

The antibodies used in the present invention are usually not particularly limited as long as they bind to a desired antigen, and may be polyclonal antibodies or monoclonal antibodies.

Monoclonal antibodies used in the present invention include not only monoclonal antibodies derived from animals such as humans, mice, rats, hamsters, rabbits, sheep, camels and monkeys, but also artificially-modified recombinant antibodies such as chimeric antibodies, humanized antibodies, bispecific antibodies. Furthermore, recombinant antibodies where an antibody's constant region and the like are artificially modified in order to alter the physical properties of antibody molecules for the purpose of improving blood retention and *in vivo* kinetics (specifically, to modify the isoelectric point (pI), affinity to Fc receptors, *etc.*) are also included.

The immunoglobulin class of the antibodies used in the present invention is not particularly limited, and IgGs such as IgG1, IgG2, IgG3, and IgG4 may be used. Preferred IgGs in the present invention are IgG1, IgG2, and IgG4, especially if the Fc region is of human origin.

The antibodies used in the present invention can also be used as pharmaceutical compositions, and can be administered using any known method including parenteral administration, intrapulmonary administration, and nasal administration, and if desired for topical treatment, intralesional administration. Parenteral injections include intramuscular, intravenous, intraperitoneal, and subcutaneous administration.

When an antibody used in the present invention is used as a pharmaceutical composition, a product that contains the pharmaceutical composition and equipment useful for treatment, prevention, and/or diagnosis is provided. The product includes a container, a label on the container and a package insert attached to the container. Preferred containers include, for example, bottles, vials, syringes, IV solution bags, and the like. Containers may be made of various materials such as glass and plastic, and silicon-free syringes and the like can also be used.

The antibodies used in the present invention described above can be produced by a method well known in the art. A hybridoma that produces a monoclonal antibody can be produced as follows, basically using a known technique. More specifically, a desired antigen or cells expressing a desired antigen is/are used as a sensitizing antigen, which is used for immunization according to a normal immunization method, and the obtained immune cells are fused with known parent cells by a normal cell fusion method, and a monoclonal antibody can be produced by screening for monoclonal antibody-producing cells (hybridomas) by a conventional screening method. The hybridoma can be produced, for example, according to the method of Milstein et al. (Kohler, G. and Milstein, C., Methods Enzymol. (1981) 73: 3-46) or such. When the immunogenicity of the antigen is low, the immunization can be done by coupling the antigen to a macromolecule having immunogenicity such as albumin.

In addition, it is possible to use a recombinant antibody produced by cloning an antibody gene from a hybridoma, inserting it into an appropriate vector, introducing it into a host, and producing it using a gene recombination technique (see, for example, Carl, A.K. Borrebaeck, James, W. Larrick, THERAPEUTIC MONOCLONAL ANTIBODIES, Published in the United Kingdom by MACMILLAN PUBLISHERS LTD., 1990). Specifically, cDNA of the variable region (V region) of the antibody is synthesized from the mRNA of the hybridoma using a reverse transcriptase. Once the DNA encoding the V region of the antibody of interest is obtained, it is ligated with a DNA encoding the desired antibody constant region (C region) and inserted into an expression vector. Alternatively, the DNA encoding the V region of the antibody may be inserted into an expression vector containing the DNA of the antibody C region. They are inserted into an expression vector so that it is expressed under the control of an expression control region, for example, an enhancer or a promoter. The host cells can then be transformed with this expression vector to express the antibody.

In the present invention, a recombinant antibody that has been artificially modified to reduce heterologous antigenicity to humans or such, for example, a chimeric antibody, a humanized antibody, or the like can be used. These modified antibodies can be produced using known methods. A chimeric antibody is an antibody consisting of the variable regions of the heavy and light chains of a non-human mammal antibody, for example, a mouse antibody, and the constant regions of the heavy and light chains of a human antibody. The antibody can be obtained by ligating a DNA encoding the variable region of the mouse antibody with a DNA encoding the constant region of the human antibody, inserting the ligated DNA into an expression vector, introducing into a host to produce the antibody therein.

A humanized antibody, also called a reshaped human antibody, is obtained by transplanting the complementarity determining regions (CDRs) of a non-human mammal antibody, such as a mouse antibody, into the complementarity determining regions of a human antibody, and the general gene recombination technique for this is also known. Specifically, a DNA sequence designed to connect the CDRs of the mouse antibody and the framework regions (FRs) of the human antibody is synthesized by the PCR method from several oligonucleotides prepared so as to have overlapping portions at their terminal portions. The resulting DNA is ligated with a DNA encoding the human antibody constant region, inserted the DNA into an expression vector, and introduced into a host for antibody production (see EP 239400, WO 96/02576). The FRs of the human antibody linked *via* CDRs are selected so that the complementarity determining regions form a good antigen-binding site. If desired, the amino acids in the framework regions of the variable regions of the antibody may be substituted so that the complementarity determining regions of the reshaped human antibody form the appropriate antigen-binding site (Sato, K. et al., Cancer Res. (1993) 53, 851-856).

The following techniques are known as examples for substituting amino acids of an antibody in order to improve the activity, physical properties, pharmacokinetics, safety, and such of the antibody, and the antibodies used in the present invention also include such antibodies with amino acid substitutions (including deletions and additions).

The following have been reported as techniques for substituting amino acids in the variable regions of IgG antibodies:

humanization (Tsurushita N, Hinton PR, Kumar S, Design of humanized antibodies: from anti-Tac to Zenapax., Methods. 2005 May; 36(1): 69-83.);
affinity maturation by amino acid substitutions of complementarity determination regions (CDRs) to enhance binding activity (Rajpal A., Beyaz N, Haber L, Cappuccilli G, Yee H, Bhatt RR, Takeuchi T, Lerner RA, Crea R, A general method for greatly improving the affinity of antibodies by using combinatorial libraries., Proc Natl Acad Sci USA. 2005 Jun 14; 102(24): 8466-71.); and
improvement of physicochemical stability by amino acid substitutions in frameworks (FRs) (Ewert S, Honegger A, Pluckthun A., Stability improvement of antibodies for extracellular and intracellular applications: CDR grafting to stable frameworks and structure-based framework engineering., Methods. 2004 Oct; 34(2): 184-99. Review).

Further, as techniques for substituting amino acids in the Fc region of an IgG antibody, techniques that enhance antibody-dependent cellular cytotoxicity (ADCC) activity and/or complement-dependent cellular cytotoxicity (CDC) activity are known (Kim SJ, Park Y, Hong HJ., Antibody engineering for the development of therapeutic antibodies., Mol Cells. 2005 Aug 31; 20(1): 17-29. Review.). Also reported is a technique for amino acid substitutions in the Fc region, which not only enhances such effector functions but also improves the blood half-life of antibodies (Hinton PR, Xiong JM, Johlfs MG, Tang MT, Keller S, Tsurushita N, An engineered human IgG1 antibody with longer serum half-life., J. Immunol. 2006 Jan 1; 176(1): 346-56.; Ghetie V, Popov S, Borvak J, Radu C, Matesoi D, Medesan C, Ober RJ, Ward ES, Increasing the serum persistence of an IgG fragment by random mutagenesis., Nat Biotechnol. 1997 Jul; 15(7): 637-40.). Furthermore, various amino acid substitution techniques in the constant region for the purpose of improving the physical properties of antibodies are also known (WO 09/41613).

Methods for obtaining human antibodies are also known. For example, it is possible to obtain a desired human antibody with binding activity to an antigen by immunizing human lymphocytes *in vitro* with the desired antigen or cells expressing the desired antigen, and fusing the immunized lymphocytes with human myeloma cells, such as U266 (see JP-A (*Kohyo*) H01-59878). In addition, a desired human antibody can be obtained by immunizing transgenic animals having the complete repertoire of human antibody genes with an antigen (see WO 93/12227, WO 92/03918, WO 94/02602, WO 94/25585, WO 96/34096, WO 96/33735). Technologies for obtaining a human antibody by panning using a human antibody library are also known. For example, a phage that binds to an antigen can be selected by expressing the variable region of a human antibody as a single-chain antibody (scFv) on the surface of the phage by the phage display method. By analyzing the genes of the selected phage, the DNA sequence encoding the variable region of the human antibody that binds to the antigen can be determined. Once the DNA sequence of scFv that binds to the antigen is clarified, a suitable expression vector containing the sequence can be prepared and the human antibody can be obtained. These methods are already well known and WO 92/01047, WO 92/20791, WO 93/06213, WO 93/11236, WO 93/19172, WO 95/01438, WO 95/15388 can be referred to. Antibodies used in the present invention also include such human antibodies.

When an antibody gene is once isolated and introduced into a suitable host to prepare an antibody, a suitable combination of host and an expression vector can be used. When eukaryotic cells are used as host, animal cells, plant cells, and fungal cells can be used. Animal cells including (1) mammalian cells, for example, CHO, COS, myeloma, BHK (baby hamster kidney), HeLa, Vero; (2) amphibian cells, for example, Xenopus oocytes; and (3) insect cells, for example, sf9, sf21, Tn5, and such are known. As plant cells, cells derived from the genus *Nicotiana,* for example, *Nicotiana tabacum,* are known, and these cells may be callus cultured. Known fungal cells include yeasts such as the genus *Saccharomyces,* for example, *Saccharomyces cerevisiae,* and filamentous fungi, such as the genus *Aspergillus,* for example, *Aspergillus niger.* When using prokaryotic cells, there are production systems that use bacterial cells. *E. coli* and *Bacillus subtilis* are known as bacterial cells. Antibodies can be obtained by introducing the target antibody genes into these cells by transformation and culturing the transformed cells *in vitro.*

Antibodies linked to various molecules such as polyethylene glycol (PEG) and cytotoxic agents can also be used as antibody modification products (Farmaco. 1999 Aug 30; 54(8): 497-516., Cancer J. 2008. May-Jun; 14(3): 154-69.). These antibody modification products are also encompassed by the antibodies used in the present invention. Such antibody modification products can be obtained by chemically modifying an antibody. These methods have already been established in this field.

Antibodies used in the present invention include anti-tissue factor antibodies, anti-IL-6 receptor antibodies, anti-IL-6 antibodies, anti-HM1.24 antigen monoclonal antibodies, anti-parathyroid hormone-related peptide antibodies (anti-PTHrP antibodies), anti-glypican-3 antibodies, anti-ganglioside GM3 antibodies, anti-TPO receptor agonist antibodies, coagulation Factor VIII function-substituting antibodies, anti-IL31 receptor antibodies, anti-HLA antibodies, anti-AXL antibodies, anti-CXCR4 antibodies, anti-NRIO antibodies, and bispecific antibodies that recognize Factor IX(a) and Factor X, but are not limited thereto.

Further, the pI values of the antibodies used in the present invention are preferably from 4.0 to 10.0, more preferably from 5.0 to 9.5, and still more preferably from 6.0 to 9.0. The pI values are elevated compared to the pI value of the IgG antibody before the amino acid modification. The isoelectric point of an IgG antibody or the like can be evaluated by a known analysis method such as isoelectric focusing.

The amount of binding between a Protein A-modified ligand in the present invention and the Fc region of the IgG antibody to be purified in the present invention is preferably 5 times or more, more preferably 10 times or more, as compared to the amount of binding to unmodified Protein A. The binding amount referred to here is not particularly limited to the method of measurement, and an example thereof includes the method of measuring the dynamic binding capacity described in the Examples herein.

Commonly-used Protein A columns specifically include, for example, HiTrap MabSelect SuRe (manufactured by GE Healthcare, trade name), Amsphere A3 (manufactured by JSR Life Sciences, registered trademark), MiniChrom Column Eshmuno A (manufactured by Merck Millipore, registered trademark), MabSpeed rP202 (manufactured by Mitsubishi Chemical, registered trademark), and KanCap Pre-packaged Column (manufactured by Kaneka, trade name).

The Protein A affinity column used in the present invention includes an affinity column containing a carrier onto which a Protein A-modified ligand has been immobilized, wherein the Protein A-modified ligand comprises: a modified immunoglobulin-binding domain comprising a modification for substitution of any one or more originally present lysine residues at positions 4, 7, and 35 of the C-domain variant or Z-domain with amino acid residues other than lysine, and also has a binding ability to an IgG antibody comprising the amino acid residue substitutions Q311R and R343R; or a multimer of these modified immunoglobulin-binding domains. This Protein A-modified ligand is characterized in that, when immobilized onto an insoluble carrier *via* its own amino groups, the orientation for maintaining the affinity for immunoglobulin is improved as compared with the unmodified molecule, through the modification of substitution for any one or more lysine residues at positions 4, 7 and 35 of the C-domain variant (SEQ ID NO: 1) or Z-domain (SEQ ID NO: 2) with other amino acid residues, the C-domain variant being a domain in which the glycine residue at position 29 of the amino acid sequence of the C-domain of Protein A has been substituted with an alanine residue.

In the present invention, the "immunoglobulin-binding domain" refers to a functional unit of a polypeptide having an immunoglobulin-binding activity by itself, and the "modified immunoglobulin-binding domain" is a domain in which a modification has been added to the original immunoglobulin-binding domain. The "ligand" refers to a molecule having the property of binding to a specific molecule by a specific affinity, and in the present invention, refers to an immunoglobulin-binding protein that can selectively bind to immunoglobulins. The "Protein A-modified ligand" refers to an immunoglobulin-binding protein comprising a modified immunoglobulin-binding domain in which the binding domain of Protein A has been modified. Here, in the present specification, the "modified immunoglobulin-binding domain" and the "Protein A-modified ligand" are collectively referred to as "modified protein".

The modified immunoglobulin-binding domain used in the present invention can contain an amino acid sequence in which, in the C-domain variant (SEQ ID NO: 1) or in the Z-domain (SEQ ID NO: 2), one or more lysine residues at positions 4, 7, and 35 are additionally substituted with other amino acid residues. For example, of the positions 4, 7, and 35, it is desirable that two or more lysine residues, preferably three lysine residues, are substituted with other amino acid residues.

The type of amino acid after substitution at any one or more of positions 4, 7, and 35 of the C-domain variant (SEQ ID NO: 1) or Z-domain (SEQ ID NO: 2) is not particularly limited, but it is preferably alanine, glutamine, asparagine, valine, serine, threonine, histidine, tyrosine, or arginine, and more preferably alanine, threonine, or arginine.

More specifically, the type of amino acid after substitution at position 4 of the C-domain variant (SEQ ID NO: 1) or Z-domain (SEQ ID NO: 2) is preferably glutamic acid, isoleucine, arginine, alanine, valine, serine, threonine, or histidine, and more preferably alanine.

The type of amino acid after substitution at position 7 of the C-domain variant (SEQ ID NO: 1) or Z-domain (SEQ ID NO: 2) is preferably tyrosine, phenylalanine, glutamine, leucine, isoleucine, proline, threonine, alanine, valine, serine, arginine, or histidine, and more preferably threonine.

The type of amino acid after substitution at position 35 of the C-domain variant (SEQ ID NO: 1) or Z-domain (SEQ ID NO: 2) is preferably arginine, glutamine, asparagine, or tyrosine, and more preferably arginine.

In addition to the above modifications, the C-domain variant (SEQ ID NO: 1) or Z-domain (SEQ ID NO: 2) may additionally have one to several amino acids substituted to the extent that it has the ability to bind to an IgG antibody comprising the amino acid residue substitutions Q311R and R343R. In particular, in the C-domain variant (SEQ ID NO: 1) or Z-domain (SEQ ID NO: 2), it is preferred that the number of lysines contained as constituent amino acids is preferably small, and it is desirable that in addition to the above modifications, 1 to 4, preferably 3 or 4, or more preferably 4 of the originally present lysine residues at positions 42, 49, 50 and 58 are substituted with amino acid residues other than lysine.

The type of amino acid after substitution at any one or more of positions 42, 49, 50, and 58 of the C-domain variant (SEQ ID NO: 1) or Z-domain (SEQ ID NO: 2) is not particularly limited, but is preferably alanine, glutamine, asparagine, valine, serine, threonine, histidine, tyrosine, or arginine, and more preferably alanine or arginine.

More specifically, if the originally present lysine residue at position 42 of the C-domain variant (SEQ ID NO: 1) or Z-domain (SEQ ID NO: 2) is substituted with another amino acid residue, the type of amino acid after the substitution is preferably alanine, valine, serine, threonine, or histidine, and more preferably alanine.

If the originally present lysine residue at position 49 of the C-domain variant (SEQ ID NO: 1) or Z-domain (SEQ ID NO: 2) is substituted with another amino acid residue, the type of amino acid after the substitution is preferably arginine, glutamine, asparagine, or tyrosine, and more preferably arginine.

If the originally present lysine residue at position 50 of the C-domain variant (SEQ ID NO: 1) or Z-domain (SEQ ID NO: 2) is substituted with another amino acid residue, the type of amino acid after the substitution is preferably arginine, glutamine, asparagine, or tyrosine, and more preferably arginine.

If the originally present lysine residue at position 58 of the C-domain variant (SEQ ID NO: 1) or Z-domain (SEQ ID NO: 2) is substituted with another amino acid residue, the type of amino acid after the substitution is preferably arginine, glutamine, asparagine, or tyrosine, and more preferably arginine.

Furthermore, in the modified immunoglobulin-binding domain in the present invention, in addition to the above modifications, the aspartic acid residue at position 37 of the C-domain variant (SEQ ID NO: 1) or Z-domain (SEQ ID NO: 2) may be substituted with an amino acid residue other than aspartic acid. This modification improves the chemical stability under acidic pH conditions as compared to an unmodified molecule.

If the original aspartic acid residue at position 37 of the C-domain variant (SEQ ID NO: 1) or Z-domain (SEQ ID NO: 2) is substituted with another amino acid residue, the type of amino acid after the substitution is not particularly limited, but alanine, glutamic acid, serine, threonine, leucine, or isoleucine is preferred.

A suitable example of the amino acid sequence of the modified immunoglobulin-binding domain used in the present invention includes the amino acid sequence of SEQ ID NO: 3 or 5. The amino acid sequence of SEQ ID NO: 3 is an amino acid sequence in which position 4 in the amino acid sequence of SEQ ID NO: 1 has been substituted with an alanine residue, position 7 with a threonine residue, and position 35 with an arginine residue. The amino acid sequence of SEQ ID NO: 5 is an amino acid sequence in which position 4 in the amino acid sequence of SEQ ID NO: 1 has been substituted with an alanine residue, position 7 with a threonine residue, position 35 with an arginine residue, position 42 with an alanine residue, position 49 with an arginine residue, position 50 with an arginine residue, and position 58 with an arginine residue.

Alternatively, in addition to the above sequences, suitable examples of amino acid sequences of the modified immunoglobulin-binding domain in the present invention can include the following amino acid sequences:

[1] in the C-domain variant defined by SEQ ID NO: 1, the lysine at position 35 is substituted with glutamine or arginine;
[2] in the C-domain variant defined by SEQ ID NO: 1, the amino acid residues at positions 40, 43, 46, and 53 are substituted with lysine, and the lysine at position 35 is substituted with arginine or valine;
[3] additionally in the amino acid sequence of [2], the lysine at position 7 is substituted with tyrosine, phenylalanine, threonine, arginine, glutamine, valine, leucine, isoleucine, histidine, alanine, or proline;
[4] additionally in the amino acid sequence of [2] or [3], the lysine at position 4 is substituted with alanine;
[5] in the C-domain variant defined by SEQ ID NO: 1, the amino acid residues at positions 40, 43, 46, and 53 are substituted with lysine, and the lysine at position 4 is substituted with valine, isoleucine, glutamic acid, or arginine;
[6] in the C-domain variant defined by SEQ ID NO: 1, the lysine at position 42 is substituted with alanine, the lysines at positions 49, 50, and 58 are substituted with arginine, and additionally the lysine at position 4 is substituted with valine, isoleucine, glutamic acid or arginine; and
[7] additionally in the amino acid sequence of [5] or [6], the lysines at positions 7 and 35 are substituted with arginine.

When the Protein A-modified ligand is a multimer having an immunoglobulin-binding domain as the constituent unit, it is sufficient to contain one or more immunoglobulin-binding domains comprising the above modification(s). The number of units of the immunoglobulin binding domain contained in the multimer is, for example, 2 to 10, preferably 2 to 8, more preferably 4 to 7, and even more preferably 6. The multimer may contain immunoglobulin-binding domains that do not contain the above-mentioned modification(s), as long as it contains one or more immunoglobulin-binding domains that contain the above-mentioned modification(s). In the multimer, the ratio of immunoglobulin-binding domains containing the above-mentioned modification(s) to the total number of immunoglobulin-binding domains contained as constituent units is preferably 50% or more, and more preferably 100% (that is, all of the immunoglobulin-binding domains contained as constituent units have the above-mentioned modification(s)).

When the Protein A-modified ligand is a multimer having an immunoglobulin-binding domain as the constituent unit, the immunoglobulin-binding domain arranged at the first and/or second from the N-terminal or C-terminal side of the multimer may be substituted with an amino acid residue that can covalently bind to the carrier (e.g., lysine residue) to facilitate immobilization onto the carrier. For example, immobilization onto the carrier becomes easier when at least 1, preferably 2 to 6, more preferably 3 or 4, and even more preferably 4 originally present amino acid residues from among those at positions 40, 43, 46, 53, 54, and 56 in the C-domain variant (SEQ ID NO: 1) or Z-domain (SEQ ID NO: 2) that is arranged at the first and/or second from the N-terminal or C-terminal side of the multimer are substituted with lysine residues.

Further, in the immunoglobulin-binding domain arranged at the first and/or second from the N-terminal or C-terminal side of the multimer, one or more lysine residues at positions 4, 7, and 35 in the C-domain variant (SEQ ID NO: 1) or Z-domain (SEQ ID NO: 2) may not be substituted with other amino acid residues, but one or more of these lysine residues may be substituted with other amino acid residues. An example of an immunoglobulin-binding domain arranged at the first and/or second from the N-terminal or C-terminal side of the multimer includes the amino acid sequence of SEQ ID NO: 4. In the amino acid sequence of SEQ ID NO: 4, position 4 in the amino acid sequence of SEQ ID NO: 1 has been substituted with an alanine residue, position 7 with a threonine residue, position 35 with an arginine residue, position 40 with a lysine residue, position 43 with a lysine residue, position 46 with a lysine residue, and position 53 with a lysine residue.

An example of the above multimer includes a modified Protein A ligand represented by the following Formula (1).

$$(R1)_n\text{-}(R2)_m \text{ or } (R2)_m\text{-}(R1)_n \qquad (1)$$

In Formula (1), the left end is the N-terminus and the right end is the C-terminus.
In Formula (1), "n" is an integer of 1 or more and 9 or less, preferably an integer of 1 or more and 7 or less, more preferably an integer of 3 or more and 6 or less, and further preferably 5. "m" is an integer of 1 or 2, and preferably 1. The total number of domains "n + m" is 2 to 10, preferably 2 to 8, more preferably 4 to 7, and further preferably 6.

In Formula (1), (R1) is a modified immunoglobulin-binding domain in which one or more (preferably all) lysine residues at positions 4, 7, and 35 of the C-domain variant (SEQ ID NO: 1) or Z-domain (SEQ ID NO: 2) have been substituted with amino acid residues other than a lysine residue. It is preferred that in (R1), in addition to the substitutions at positions 4, 7, and 35 mentioned above, any one or more (preferably all) lysine residues at positions 42, 49, 50, and 58 have been substituted with amino acid residues other than lysine. The "n" number of (R1) may all have the same amino acid sequence, or may have amino acid sequences different from each other.

In Formula (1), (R2) is an immunoglobulin-binding domain in which one or more (preferably all) amino acid residues at positions 40, 43, 46, 53, 54, and 56 of the C-domain variant (SEQ ID NO: 1) or Z-domain (SEQ ID NO: 2) have been substituted with a lysine residue. It is preferred that in (R2), in addition to any one or more of the substitutions at positions 40, 43, 46, 53, 54, and 56, any one or more (preferably all) lysine residues at positions 4, 7, and 35 have been substituted with amino acid residues other than lysine. When "m" is 2, the two (R2) may both have the same amino acid sequence, but may also have different sequences from each other.

A suitable example of the modified Protein A ligand represented by the above Formula (1) includes "n" is 5; "m" is 1; (R1) is a modified immunoglobulin-binding domain consisting of the amino acid sequence of SEQ ID NO: 5 (an amino acid sequence where position 4 in SEQ ID NO: 1 is substituted with an alanine residue, position 7 with a threonine residue, position 35 with an arginine residue, position 42 with an alanine residue, position 49 with an arginine residue, position 50 with an arginine residue, and position 58 with an arginine residue); and (R2) is a modified immunoglobulin-binding domain consisting of the amino acid sequence of SEQ ID NO: 4 (an amino acid sequence where position 4 in SEQ ID NO: 1 is substituted with an alanine residue, position 7 with a threonine residue, position 35 with an arginine residue, position 40 with a lysine residue, position 43 with a lysine residue, position 46 with a lysine residue, and position 53 with a lysine residue).

A Protein A-modified protein of the present invention can be produced by using known genetic engineering techniques described in, for example, Current Protocols in Molecular Biology by Frederick M. Ausbel *et al.* More specifically, the protein can be obtained from cultured cells in a large amount at a low cost by transforming a host such as *Escherichia coli* with an expression vector including a nucleotide sequence encoding a target modified protein and culturing the cells in an appropriate liquid medium. Specifically, since one immunoglobulin-binding domain of Protein A is a small protein consisting of about 60 amino acids, a target expression vector can be obtained by, for example, dividing a DNA encoding a desired amino acid sequence into synthetic oligonucleotides consisting of several tens of bases, synthesizing them, ligating them by a ligation reaction with DNA ligase, and inserting them into a plasmid vector.

At that time, for the purpose of efficiently expressing the protein in *E. coli,* one skilled in the art usually employs a nucleotide sequence using the optimum codons of *E. coli.* Further, any domain of Protein A may be employed as the amino acid sequence of an unmodified immunoglobulin-binding domain, but among the five originally existing domains, a C-domain having many lysine residues at positions 39 onwards is preferably used. Alternatively, the Z-domain sequence that has often been used as an affinity ligand for immunoglobulins may be utilized, but it is most preferable to employ the sequence of the C-domain variant (shown in SEQ ID NO: 1 in Sequence Listing) where the glycine residue at position 29 has been substituted with an alanine residue, which has already been known to increase chemical stability (Nilsson B. et. al., Protein Engineering, 1(2), pp. 107-113). Mutations in the DNA sequence for achieving the target amino acid substitutions can be easily introduced into intended sites by using a method such as the overlap extension method using an unmodified clone DNA as a template and using, as primers, synthetic oligo DNAs which incorporate mismatched base pairs for the polymerase chain reaction, and the cassette mutation method. Furthermore, in the case of using a Protein A-derived immunoglobulin-binding protein as an affinity chromatography ligand for an immunoglobulin, a multimeric protein obtained by ligating two or more, desirably about four immunoglobulin-binding domains has been conventionally produced and used. For the immunoglobulin-binding protein obtained by the present invention, it is preferable to produce and use a multimeric protein obtained by ligating two or more, preferably two to ten, more preferably four to seven, and even more preferably six immunoglobulin-binding domains. A cDNA encoding such a multimeric protein can be easily prepared by linking the intended number of cDNAs each encoding one immunoglobulin-binding domain in series. A multimeric protein in which two or more of immunoglobulin-binding domain units are linked can be easily produced by using thus prepared cDNA inserted into an appropriate expression plasmid.

Any vectors such as plasmids, phages, or viruses that can replicate itself in host cells can be used as the expression vector to be inserted with a nucleotide sequence encoding the modified protein of the present invention. For example, commercially available expression vectors include pQE system vectors (QIAGEN), pDR540, pRIT2T (GE Healthcare Bioscience Co., Ltd.), pET system vectors (Merck Co., Ltd.). The expression vector is preferably used by selecting an appropriate combination with the host cell. For example, when *E. coli* is used as a host cell, preferred examples include a combination of a pET system vector and the BL21 (DE3) *E. coli* strain and a combination of the pDR540 vector and the JM109 *E. coli* strain.

The modified protein of the present invention can be recovered in a soluble fraction by collecting cultured cells by centrifugation or the like and homogenizing them by a treatment using ultrasonic waves, French press, or the like. Purification of the modified protein can be performed by appropriately combining known separation/purification techniques. Specifically, techniques include separation techniques such as the salting-out, dialysis, and ultrafiltration; and purification methods such as hydrophobic chromatography, gel filtration chromatography, ion exchange chromatography, affinity chromatography, and reverse-phase chromatography.

Examples of an insoluble carrier for binding to the modified protein of the present invention as an affinity ligand for immunoglobulin include natural polymer materials such as chitosan and dextran, and synthetic polymers such as vinyl polymers, highly crosslinked agarose, and polyimide. In another embodiment, the carrier may be inorganic carriers such as silica. In general, a ligand protein is immobilized onto a carrier with a coupling agent such as cyanogen bromide, epichlorohydrin, N-hydroxy succinimide, tosyl/tresyl chloride, carbodiimide, glutaraldehyde, hydrazine, or a carboxyl- or thiol-activated carrier. Such coupling reactions are well known in the art and are widely described in literatures (for example, Jansson, J.C. and Ryden, L., "Protein purification", 2nd Edition, pp. 375-442, ISBN 0-471-18626-0). The ligand protein of the present invention is characterized in that the protein binds to a carrier *via* a plurality of amino groups arranged so that orientation of the ligand can be spatially controlled. For immobilization of the protein, a carrier having an active group that can form a covalent bond by a reaction with an amino group of the protein, such as a tresyl group, an epoxy group, a carboxyl group, and a formyl group can be used. Examples of commercially available carriers include TOYOPEARL AF-Tresyl-650, TOYOPEARL AF-Epoxy-650, TOYOPEARL AF-Carboxy-650, TOYOPEARL AF-Formyl-650 (all from Tosoh Corporation), NHS-activated Sepharose, cyanogen bromide-activated Sepharose, and epoxy-activated Sepharose (all from GE Healthcare Bioscience Co., Ltd.).

As the Protein A affinity column used in the present invention, the above Protein A-modified ligand may be immobilized by any means. For example, it can be immobilized by the following means:

(1) a method of additionally substituting 1 to 6 of the amino acid residues at positions 40, 43, 46, 53, 54 and 56 in the C-domain variant or Z-domain of Protein A with a lysine residue and immobilizing it onto the carrier through the substituted lysine residue(s);
(2) a method of immobilization onto the carrier through a disulfide bond or a thioether bond by introducing cysteine into the C-terminus of Protein A;
(3) a method of immobilization onto an amino group-containing immobilization carrier by cyanation of a thiol group;
(4) a method of immobilizing a multimer of modified immunoglobulin-binding domains having a cysteine residue onto an amino group-containing carrier using 4-(N-maleimidomethyl)cyclohexane-1-carboxylate (SMCC) as a cross-linking agent; and
(5) a method of immobilization onto the carrier through a plurality of (for example, five) lysine residues added to the C-terminus of a modified immunoglobulin-binding domain in which lysine residues at positions 42, 49, 50, and 58 of the C-domain variant of Protein A have been substituted with amino acids other than lysine, or to the C-terminus of a modified immunoglobulin-binding domain in which lysine residues at positions 49, 50, and 58 of the Z-domain of Protein A with amino acids other than lysine.

The above immobilization methods can be carried out by the usual methods.

Preferred method is the method of additionally substituting 1 to 6 of the amino acid residues at positions 40, 43, 46, 53, 54 and 56 in the C-domain variant or Z-domain of Protein A with a lysine residue and immobilizing it onto the carrier through the substituted lysine residue(s).

The Protein A affinity columns used in the present invention specifically include AF-rProtein A HC-650F (manufactured by Tosoh), which is an affinity resin in which an Fc-binding ligand (recombinant of modified Protein A) has been coupled to the synthetic polymer carrier TOYOPEARL HW-56.

The thus prepared carrier onto which a Protein A-modified ligand has been immobilized can be packed in a column to prepare an affinity column (step a). Next, the prepared affinity column is loaded with a composition containing an IgG antibody comprising modified amino acid sequence in the CH2 region and CH3 region (step b). In the present invention, the composition containing an IgG antibody refers to, for example, a culture of IgG antibody-expressing cells or supernatants thereof. In general, a culture constitutes a complex composition composed of various components such as metabolites of cells as well as various nutrients necessary for culturing. In order to highly purify the target IgG antibody to the purity required for pharmaceutical raw materials from that, it is necessary to determine the purification conditions suitable for the target IgG antibody. The present invention revealed that, to purify an IgG antibody comprising the amino acid residue substitutions Q311R and P343R in the CH2 region and CH3 region, a variant comprising a modification for substitution of any one or more originally present lysine residues at positions 4, 7, and 35 of the C-domain or Z-domain of Protein A with amino acid residues other than lysine and having an ability to bind to the IgG antibody is a suitable purification tool.

The composition containing the IgG antibody can also be pretreated by filtration, centrifugation or the like before being loaded onto the affinity column. The composition containing the IgG antibody can be loaded onto the affinity column by a general liquid chromatography system at an appropriate pressure and flow rate depending on the size and volume of the column, the size of the carrier, and the like. It is desirable that the amount of the IgG antibody composition loaded onto the affinity column is about the same as the IgG antibody binding capacity of the affinity column. For example, the binding capacity of the IgG antibody can be determined by monitoring the concentration of the IgG antibody flowing through from the affinity column loaded with the composition. More specifically, when the level of the IgG antibody flowing through from the affinity column becomes the same level as the IgG antibody concentration in the loaded composition, it can be determined that the affinity column condition is close to the IgG antibody binding capacity of the affinity column. Efficient purification of the IgG antibody can be expected by then eluting the IgG antibody from the affinity column (step c).

The purification methods of the present invention may additionally comprise the step of washing the affinity column with a washing solution before step (c).

The washing solution is not particularly limited, but the following solution can be used: a combination of a buffer and a salt, which comprises, as a buffer, at least one selected from the group consisting of phosphoric acid, acetic acid, citric acid, glycine, and tris hydroxymethyl aminomethane, and as the salt, at least one selected from the group consisting of arginine, sodium chloride and sodium sulfate.

Purified IgG antibody can be recovered by eluting the IgG antibody adsorbed onto the affinity column (step c), after, as necessary, washing the affinity column. The method of eluting the IgG antibody adsorbed onto the Protein A-modified ligand can be appropriately selected from known conditions. For example, a solution containing at least one selected from the group consisting of hydrochloric acid, acetic acid, citric acid, arginine, glycine or phosphoric acid can be utilized. The concentration of the solution for eluting the IgG antibody from the affinity column can be adjusted as appropriate according to the purpose, and can be, for example, for acetic acid, 20 mM to 500 mM, and usually 50 mM to 200 mM, and for hydrochloric acid, 1 mM to 5 mM. Even while the IgG antibody is eluted from the affinity column, the IgG antibody elution can be traced by monitoring the protein concentration in the eluate.

After step (c), the recovered IgG antibody can be further purified if necessary. For example, the purification methods of the present invention may additionally include a step(s) of purifying the IgG antibody by at least one chromatography selected from the group consisting of cation exchange chromatography, anion exchange chromatography, hydrophobic interaction chromatography, multimode chromatography and hydroxyapatite chromatography.

Through the above steps, in a preferred embodiment, the present invention can isolate an IgG antibody from within or outside (medium, *etc.*) of host cells and purify it as a substantially pure and homogeneous IgG antibody. More specifically, the present invention provides a method of producing a purified IgG antibody, the method comprising the following steps of:

> (i) providing a composition containing an IgG antibody comprising the amino acid residue substitutions Q311R and P343R;
> (ii) preparing an affinity column containing a carrier onto which a Protein A-modified ligand is immobilized, wherein the Protein A-modified ligand comprises a modified immunoglobulin-binding domain containing a modification for substitution of any one or more originally present lysine residues at positions 4, 7, and 35 of the C-domain variant of *Staphylococcus* protein A of SEQ ID NO: 1 or Z-domain of *Staphylococcus* Protein A of SEQ ID NO: 2 with amino acid residues other than lysine or a multimer of these modified immunoglobulin-binding domains;
> (iii) loading the composition containing the IgG antibody onto the affinity column of step (ii); and
> (iv) eluting and recovering the IgG antibody from the affinity column loaded with the composition containing the IgG antibody of step (iii).

The present invention also encompasses highly purified IgG antibodies using the purification methods.

The present invention will be specifically described below with reference to examples, but is not limited thereto.

[Example 1]

An Antibody A has an increased isoelectric point (pI) and enhanced affinity for Fc receptor IIb (FcRIIb) and neonatal Fc receptors (FcRn) through antibody engineering techniques to improve its pharmacokinetics, which comprises the amino acid residue substitutions of Q311R and P343R in the CH2 and CH3 regions and has the Fc region of SEQ ID NO: 10; however, as a result, it has a weakened binding affinity for Protein A. In other words, although Antibody A has improved its usefulness as a drug by having improved pharmacokinetics, it has new production problems.

The binding affinity of an antibody to Protein A is often used in its purification process (affinity purification). Specifically, the method comprising the step of adsorbing an antibody on a column onto which Protein A has been immobilized, eluting the antibody after washing, and recovering the antibody is widely used as a method for antibody purification. Since Protein A binds to the Fc region of an antibody, it is used for purification of a wide range of antibodies regardless of the antigen specificity of the antibody. Various Protein A columns where the method for immobilizing Protein A, resin, or Protein A itself is modified are commercially available.

In order to find a Protein A-immobilized resin that can be applied to the affinity purification of Antibody A, the affinity of the antibody for the following commercially available Protein A-immobilized resin was compared:

HiTrap MabSelect SuRe (manufactured by GE Healthcare, product name);
ToyoScreen AF-rProtein A HC-650F (manufactured by Tosoh, product name);
Amsphere A3 (manufactured by JSR Life Sciences, registered trademark);
MiniChrom Column Eshmuno A (manufactured by Merck Millipore, registered trademark);
MabSpeed rP202 (manufactured by Mitsubishi Chemical Corporation, registered trademark); and
KanCap Pre-packaged Column (manufactured by Kaneka, product name).

(1) Dynamic binding capacity (DBC) of Antibody A in each column

Purified Antibody A was dissolved in equilibration buffer and loaded onto a column filled with each Protein A-immobilized resin. The protein concentration of the buffer eluted from the column was traced by ultraviolet light to identify the 5% breakthrough point, and the DBC per 1 L of resin was determined by the following formula. The 5% breakthrough point means the amount of protein loaded onto the column when the protein concentration in the eluate exceeds 5% of the protein concentration in the antibody solution loaded in the column.

$$\text{DBC} = \frac{\text{Antibody A concentration (g/L) x loaded liquid volume (5\% breakthrough point) (L)}}{\text{Column capacity (L)}}$$

Similarly, for comparison, the DBC of each column was determined for a humanized antibody that did not contain modifications in the Fc region of human IgG1.

(2) Dynamic binding capacity; DBC

[Table 1]
DBC of Antibody A in each Protein A-immobilized Resin (g/L res
i n)

| Resin | DBC |
| --- | --- |
| AF-rProtein A HC-650F | 49.1 |
| Amsphere A3 | 13.6 |
| MabSpeed rP202 | 6.4 |
| KanCap A | 2. 5 |
| Eshmuno A | 2. 3 |
| MabSelect SuRe | 1.9 |
| MabSelect SuRe LX | 1.6 |

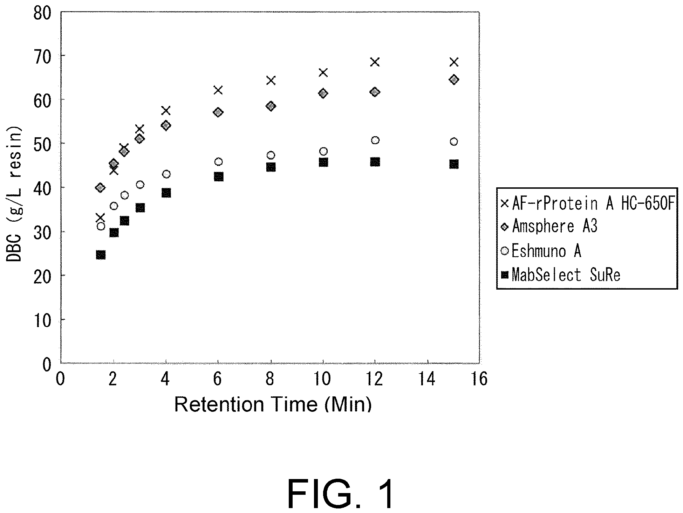

AF-rProtein A HC-650F, which had a large amount of Antibody A bound, showed high DBCs of 46.6 and 45.2 when evaluated also with two resins of different production lots. The next best DBC to AF-rProtein A HC-650F was Amsphere A3 at 13.6. The DBCs of other resins were in the range of 1.6 to 6.4, which are fairly low values. On the other hand, in an antibody in which the Fc region was not modified, the DBCs of each resin were in the range of 20 to 70 as shown in Fig. 1, which were sufficient values for antibody purification.

AF-rProtein A HC-650F (manufactured by Tosoh) is an affinity resin in which an Fc-binding ligand (recombinant of modified Protein A) has been linked to the synthetic polymer carrier TOYOPEARL HW-65. The ligand linked to AF-rProtein A HC-650F has the structure shown in the following Formula (1'):

$$(R1)_5\text{-}(R2)_1 \qquad (1')$$

In the above Formula (1'), the left end is the N-terminus and the right end is the C-terminus. In the above Formula (1'), (R2) is a modified immunoglobulin-binding domain (SEQ ID NO: 4) in which the amino acid sequence of the C-domain variant (SEQ ID NO: 1), which has been prepared by substituting (G29A) a part of the amino acid sequence of the C-domain of the immunoglobulin-binding domain constituting Protein A derived from *Staphylococcus aureus,* has been substituted as follows: position 4 with an alanine residue, position 7 with a threonine residue, position 35 with an arginine residue, and position 40 with a lysine residue, position 43 with a lysine residue, position 46 with a lysine residue, and position 53 with a lysine residue. The five (R1) located on the N-terminal side are modified immunoglobulin-binding domains (SEQ ID NO: 5) which, in addition the amino acid sequence of the C-domain variant with substitutions at position 4 with an alanine residue, position 7 with a threonine residue, and position 35 with an arginine residue, has substitutions at position 42 with an alanine residue, position 49 with an arginine residue, position 50 with an arginine residue, and position 58 with an arginine residue. More specifically, Formula (1') is a ligand consisting of an amino acid sequence in which five of the amino acid sequences of SEQ ID NO: 5 and one amino acid sequence of SEQ ID NO: 4 are linked from the N-terminal side.

In the above-mentioned variant, the binding to the Fc region has been further strengthened by making the modified immunoglobulin-binding domain a hexamer. AF-rProtein A HC-650F has a greatly improved Fc binding property and is an affinity resin with alkali resistance by utilizing the variant of the above structure.

The affinity resins used in the present comparative test are all products where the structure of Protein A itself and the binding mode with its carrier have been optimized, and the Fc region binding property and alkali resistance have been enhanced. It was found that all show excellent binding property to the antibody when an Fc region does not contain any modification, but when an Fc region is modified, there is a big difference in binding property to the antibody depending on the affinity resin.

[Example 2]

To evaluate the binding performance of ligands to pI-modified antibodies comprising the amino acid residue substitutions Q311R and P343R, the KD value was measured using BLItz (registered trademark) (ForteBio) evaluation system for AF-rProtein A HC-650F and MabSelect SuRe used in Example 1.

The ligand of AF-rProtein A HC-650F (structure shown in Formula (1'')) and the ligand of MabSelect SuRe were each labeled with Biotin (Lys:Biotin = 1:1) and then immobilized onto the surface of sensor chips. After equilibration treatment with a PBS solution, the antibody-containing solution was diluted with PBS (+ 0.1% BSA) buffer and added, and PBS (+ BSA) was further added to measure the dissociation reaction.

As the antibody, in addition to Antibody A, tocilizumab (hPM-1 or MRA: see International Patent Application Publication No. WO 92/19759), which is a humanized anti-interleukin 6 (IL-6) receptor antibody, was used as a comparative example. Tocilizumab, unlike Antibody A, does not comprise the amino acid residue substitutions Q311R and P343R for pI modification (a pI-unmodified antibody).

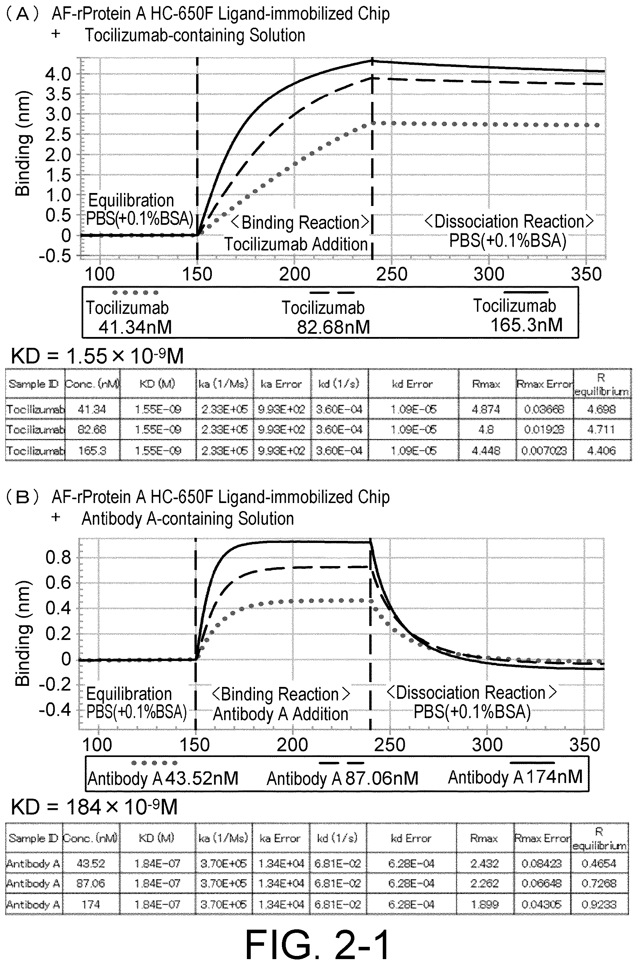

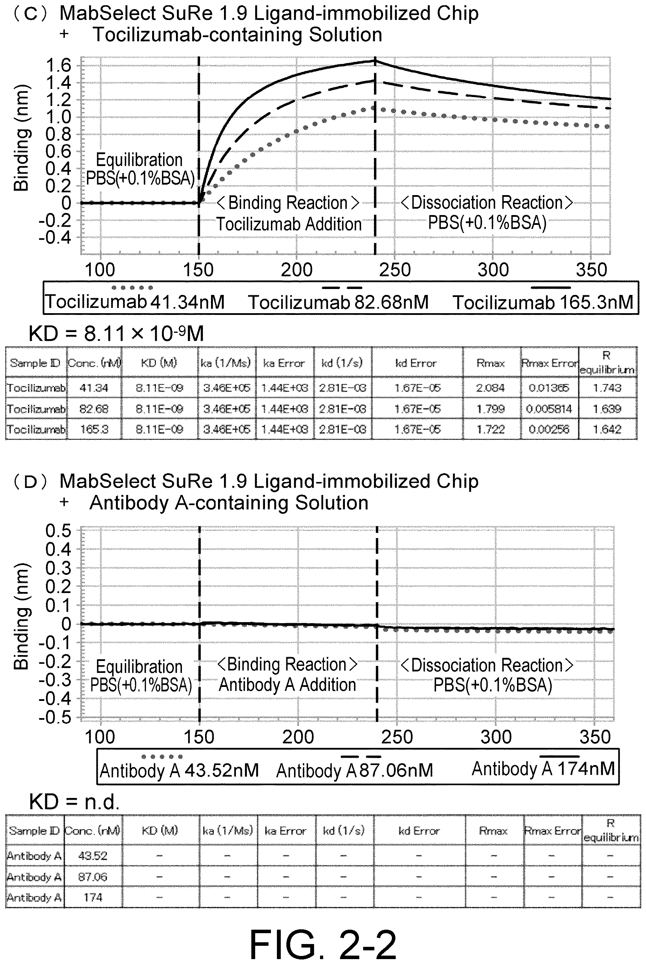

The test results are summarized in Fig. 2 and Table 2. For the AF-rProtein A HC-650F ligand, tocilizumab bound with a $K_D$ value of 1.55 x $10^{-9}$ M. In addition, Antibody A had a $K_D$ value of 184 x $10^{-9}$ M, which although had weaker affinity than tocilizumab, showed binding. On the other hand, regarding the MabSelect SuRe ligand, tocilizumab bound with a KD value of 8.11 x $10^{-9}$ M, but binding of Antibody A was not observed.

[Table 2]

| Ligand | Antibody | |
|---|---|---|
| | Tocilizumab Affinity $K_D$ ($\times 10^{-9}$ M) | Antibody A Affinity $K_D$ ($\times 10^{-9}$ M) |
| AF-rProtein A HC-650 | 1.55 | 184 |
| MabSelect SuRe | 8. 11 | n. d. |

[Example 3]

In order to evaluate the relationship between the substitution of amino acid residues of the ligand and the binding force to a pI-modified antibody, the KD value of Antibody A was measured by the same method as in Example 2 (however, changing to Lys:Biotin = 12:1), for a ligand monomer introduced with mutations at positions 4, 7, and 35 with respect to the C-domain variant (SEQ ID NO: 1).

Results of Table 3 showed that, by substituting the lysine residue (K) at position 35 of the C-domain variant with a glutamine residue (Q) or an arginine residue (R), the affinity for antibody A was increased.

[Table 3]

| Ligand | Antibody A Affinity $K_D$ ($\times 10^{-4}$ M) | Substitution to C-domain Variant (SEQ ID NO: 1) | | |
|---|---|---|---|---|
| | | Position 4 | Position 7 | Position 35 |
| C-domain Variant | n. d. | -* | - | - |
| PN61 | 1. 62 | A | T | R |
| PN34 | 1. 99 | - | - | Q |
| PN23 | 1. 16 | - | - | R |
| PN43 | n. d. | R | - | - |
| PN44 | n. d. | - | R | - |
| * "-" means that there is no substitution to the C-domain variant (position 4: K, position 7: K, position 35: K). | | | | |

Further, regarding the ligand monomer in which the amino acid residues at positions 40, 43, 46, and 53 of the C-domain variant have been substituted with a lysine residue (K) (hereinafter, "ligand monomer having the R2' structure"), different mutations were introduced at positions 4, 7, and 35, and the KD value of Antibody A was measured by the same method as in Example 2 (however, changing to Lys:Biotin = 12:1).

From the Results of Table 4, among the ligand monomers having the R2'structure, an increase in affinity for Antibody A was observed by substituting the lysine residue (K) at position 35 with an arginine residue (R) or a valine residue (V), particularly with an arginine residue (R). Furthermore, it was shown that in addition to the substitution at position 35, affinity for Antibody A was increased by substituting the lysine residue (K) at position 7 with any one of a tyrosine residue (Y), a phenylalanine residue (F), a threonine residue (T), an arginine residue (R), a glutamine residue (Q), valine residue (V), leucine residue (L), isoleucine residue (I), histidine residue (H), alanine residue (A), and proline residue (P), particularly with a tyrosine residue (Y) or a phenylalanine residue (F).

[Table 4]

| Ligand | Antibody A Affinity $K_D$ ($\times 10^{-9}$ M) | Substitution to C-domain Variant (SEQ ID NO: 1) | | | | | | |
|---|---|---|---|---|---|---|---|---|
| | | Position 4 | Position 7 | Position 35 | Position 40 | :Position 43 | Position 46 | Position 53 |
| C-domain Variant | n. d. | -* | - | - | - | - | - | - |
| R2 | 539 | A | T | R | K | K | K | K |
| PN30 | 42400 | A | - | R | K | K | K | K |
| PN133 | 80100 | A | T | H | K | K | K | K |
| PN190 | 91300 | A | T | A | K | K | K | K |
| PN12 | 39900 | - | - | - | K | K | K | K |
| PN93 | 322 | A | R | R | K | K | K | K |
| PN127 | 265 | A | Q | R | K | K | K | K |
| PN128 | 176000 | A | E | R | K | K | K | K |
| PN132 | 309 | A | V | R | K | K | K | K |
| PN194 | 257000 | A | T | E | K | K | K | K |
| PN195 | 171000 | A | T | T | K | K | K | K |
| PN196 | 1100 | A | T | V | K | K | K | K |
| PN198 | n. d. | A | T | Y | K | K | K | K |
| PN197 | 120000 | A | T | L | K | K | K | K |

(continued)

| Ligand | Antibody A Affinity $K_D$ ($\times 10^{-9}$ M) | Substitution to C-domain Variant (SEQ ID NO: 1) | | | | | | |
|---|---|---|---|---|---|---|---|---|
| | | Position 4 | Position 7 | Position 35 | Position 40 | Position 43 | Position 46 | Position 53 |
| C-domain Variant | n. d. | -* | - | - | - | - | - | - |
| PN129 | 359 | A | L | R | K | K | K | K |
| PN130 | 310 | A | I | R | K | K | K | K |
| PN131 | 314 | A | H | R | K | K | K | K |
| PN2011 | 102 | A | Y | R | K | K | K | K |
| PN2012 | 152 | A | F | R | K | K | K | K |
| PN2078 | 258 | A | A | R | K | K | K | K |
| PN2082 | 1630 | A | P | R | K | K | K | K |
| * "-" means that there is no substitution to the C-domain variant (position 4: K, position 7: K, position 35: K, position 40: V, position 43: E, position 46: A, position 53: D). | | | | | | | | |

[Example 4]

In order to evaluate the relationship between the substitution of amino acid residues of the ligand and the binding force to the pI-modified antibody, the KD value of Antibody A was measured by the same method as in Example 2 (however, changing to Lys:Biotin = 12: 1) for a plurality of ligand dimers.

The ligand dimers tested had a dimer structure of a ligand monomer having an R2'structure in which the amino acid residues at positions 40, 43, 46, and 53 have been substituted with a lysine residue (K) with respect to the C-domain variant, and a ligand monomer (hereinafter, "ligand monomer having an R1' structure") in which the amino acid residue at position 42 has been substituted with an alanine residue (A) and amino acid residues at positions 49, 50, and 58 have been substituted with an arginine residue (R) with respect to the C-domain variant, and further had mutations at positions 4, 7, and 35.

From the results of Table 5, an enhanced affinity for Antibody A was observed for all dimers in which the lysine residue (K) at position 4 was substituted with any one of a valine residue (V), an isoleucine residue (I), a glutamic acid residue (E), and an arginine residue (R).

[Table 5]

| Ligand | Antibody A Affinity $K_D$ ($\times$ $10^{-9}$ M) | | Substitution to C-domain Variant (SEQ ID NO: 1) | | | | | | | | | | |
|---|---|---|---|---|---|---|---|---|---|---|---|---|---|
| | | | Position 4 | Position 7 | Position 35 | Position 40 | Position 43 | Position 46 | Position 53 | Position 42 | Position 49 | Position 50 | Position 58 |
| PN226 | 306 | R1' | V | R | R | –* | – | – | – | A | R | R | R |
| | | R2' | V | R | R | K | K | K | K | – | – | – | – |
| PN229 | 379 | R1' | I | R | R | – | – | – | – | A | R | R | R |
| | | R2' | I | R | R | K | K | K | K | – | – | – | – |
| PN231 | 314 | R1' | E | R | R | – | – | – | – | A | R | R | R |
| | | R2' | E | R | R | K | K | K | K | – | – | – | – |
| PN232 | 258 | R1' | R | R | R | – | – | – | – | A | R | R | R |
| | | R2' | R | R | R | K | K | K | K | – | – | – | – |

* "–" means that there is no substitution for the C-domain variant (position 40: V, position 43: E, position 46: A, position 53: D, position 42: K, position 49: K, position 50: K, position 58: K).

[Example 5]

In order to evaluate the relationship between the addition of a poly-lysine residue (K) to a ligand monomer and the binding force to a pI-modified antibody, the KD value of Antibody A was measured by the same method as in Example 2 (however, changing to Lys:Biotin = 12:1).

The ligand monomer tested had a structure where five lysine residues (K) were added to the C-terminus of a ligand monomer for immobilization onto a carrier, the ligand monomer having an R1' structure in which the originally present lysine residues at positions 42, 49, 50 and 58 have been substituted with amino acids other than lysine.

From the results of Table 6, an enhancement of the affinity for Antibody A was observed by substituting the amino acid at position 35 with an arginine residue in a ligand monomer in which the originally present lysine residues at positions 42, 49, 50 and 58 have been substituted with the non-lysine, alanine residue (A) or arginine residue (R) and a plurality of lysine residues (K) have been added at the C-terminus.

[Table 6]

| Ligand | Antibody A Affinity $K_D$ ($\times10^{-9}$ M) | Substitution to C-domain Variant (SEQ ID NO: 1) | | | | | | | Addition to C-terminus |
|---|---|---|---|---|---|---|---|---|---|
| | | Position 4 | Position 7 | Position 35 | Position 42 | Position 49 | Position 50 | Position 58 | |
| PN100AH | 335 | V | R | R | A | R | R | R | Poly Lys (K) |

[Industrial applicability]

pI-modified antibodies that cannot be purified by Protein A columns commonly-used in industrial production methods can be efficiently and easily purified by using the specific Protein A affinity column based on the present invention. The present invention is useful as a stable and efficient industrial production method of therapeutic antibodies.

**Claims**

1. A method of purifying an IgG antibody comprising the amino acid residue substitutions Q311R and P343R from a composition containing the antibody, wherein the method comprises the steps of:

   (a) preparing an affinity column containing a carrier onto which a Protein A-modified ligand is immobilized, wherein the Protein A-modified ligand comprises: a modified immunoglobulin-binding domain comprising a modification for substitution of any one or more originally present lysine residues at positions 4, 7, and 35 of the C-domain variant of *Staphylococcus* Protein A of SEQ ID NO: 1 or Z-domain of *Staphylococcus* Protein A of SEQ ID NO: 2 with amino acid residues other than lysine; or a multimer of these modified immunoglobulin-binding domains;
   (b) loading the composition containing the IgG antibody onto the affinity column of step (a); and
   (c) eluting and recovering the IgG antibody from the affinity column of step (b).

2. The method of claim 1, wherein the multimer of the modified immunoglobulin-binding domains is a dimer to decamer, and wherein arranged at the first or second from the N-terminal or C-terminal side in the multimer is an immunoglobulin-binding domain in which at least one of the originally present amino acid residues at positions 40, 43, 46, 53, 54, and 56 of the C-domain variant (SEQ ID NO: 1) or Z-domain (SEQ ID NO: 2) has been substituted with a lysine residue.

3. The method of claim 1 or 2, wherein the substitution is a modification for substitution of any one or more originally present lysine residues at positions 4, 7, and 35 of the C-domain variant or Z-domain of Protein A with any one of amino acid residues selected from the group consisting of an alanine residue, a glutamine residue, an asparagine residue, a valine residue, a serine residue, a threonine residue, a histidine residue, a tyrosine residue, an arginine residue, a glutamic acid residue, a phenylalanine residue, a leucine residue, an isoleucine residue, and a proline residue.

4. The method of any one of claims 1 to 3, wherein the modified immunoglobulin-binding domain is (i) a modified immunoglobulin-binding domain comprising the amino acid sequence of SEQ ID NO: 3 or 5; or (ii) a modified immunoglobulin-binding domain comprising an amino acid sequence in which one to several amino acid residues have been substituted, deleted, added, and/or inserted to the amino acid sequence of SEQ ID NO: 3 or 5 at amino acid residues other than those at positions 4, 7, and 35.

5. The method of any one of claims 1 to 4, wherein the modified immunoglobulin-binding domain has an ability to bind to an IgG antibody comprising the amino acid residue substitutions Q311R and P343R.

6. The method of any one of claims 1 to 5, wherein the Protein A-modified ligand is a modified ligand comprising a modified immunoglobulin-binding domain that consists of at least one amino acid sequence selected from the group consisting of SEQ ID NOs: 3, 4, and 5.

7. The method of any one of claims 1 to 6, wherein the Protein A-modified ligand is immobilized onto the carrier by any one means selected from the group consisting of (1) to (5) below:

   (1) a method of immobilization onto the carrier through a modified immunoglobulin-binding domain in which 1 to 6 of the amino acid residues at positions 40, 43, 46, 53, 54, and 56 in the C-domain variant or Z-domain of Protein A are additionally substituted with a lysine residue;
   (2) a method of immobilization onto the carrier through a disulfide bond or a thioether bond by introducing cysteine into the C-terminus of Protein A;
   (3) a method of immobilization onto an amino group-containing immobilization carrier by cyanation of a thiol group;
   (4) a method of immobilizing a multimer of modified immunoglobulin-binding domains having a cysteine residue onto an amino group-containing carrier using 4-(N-maleimidomethyl)cyclohexane-1-carboxylate (SMCC) as a cross-linking agent; and
   (5) a method of immobilization onto the carrier through a plurality of lysine residues added to the C-terminus of a modified immunoglobulin-binding domain in which the lysine residues at positions 42, 49, 50, and 58 of the C-domain variant of Protein A are substituted with amino acids other than lysine, or to a modified immunoglobulin-binding domain in which the lysine residues at positions 49, 50, and 58 of the Z-domain are substituted with amino acids other than lysine.

8.  The method of any one of claims 1 to 7, wherein the IgG antibody is an IgG antibody additionally comprising one or more amino acid residue substitutions selected from among M428L, N434A, Y436T, Q438R, and S440E in the CH3 region of the IgG antibody.

9.  The method of any one of claims 1 to 8, wherein the IgG antibody is one in which the CH3 region of the IgG antibody comprises an amino acid sequence selected from the group consisting of SEQ ID NOs: 6 to 11.

10. The method of any one of claims 1 to 9, wherein the IgG antibody is one in which the heavy chain constant region of the IgG antibody comprises an amino acid sequence selected from the group consisting of SEQ ID NOs: 12 to 57.

11. The method of any one of claims 1 to 10, wherein the pI value of the antibody is 4.0 to 10.0.

FIG. 1

（A） AF-rProtein A HC-650F Ligand-immobilized Chip
　　　＋　Tocilizumab-containing Solution

Tocilizumab
41.34nM

Tocilizumab
82.68nM

Tocilizumab
165.3nM

$KD = 1.55 \times 10^{-9} M$

| Sample ID | Conc. (nM) | KD (M) | ka (1/Ms) | ka Error | kd (1/s) | kd Error | Rmax | Rmax Error | R equilibrium |
|---|---|---|---|---|---|---|---|---|---|
| Tocilizumab | 41.34 | 1.55E-09 | 2.33E+05 | 9.93E+02 | 3.60E-04 | 1.09E-05 | 4.874 | 0.03668 | 4.698 |
| Tocilizumab | 82.68 | 1.55E-09 | 2.33E+05 | 9.93E+02 | 3.60E-04 | 1.09E-05 | 4.8 | 0.01923 | 4.711 |
| Tocilizumab | 165.3 | 1.55E-09 | 2.33E+05 | 9.93E+02 | 3.60E-04 | 1.09E-05 | 4.448 | 0.007023 | 4.406 |

（B） AF-rProtein A HC-650F Ligand-immobilized Chip
　　　＋　Antibody A-containing Solution

Antibody A 43.52nM　　Antibody A 87.06nM　　Antibody A 174nM

$KD = 184 \times 10^{-9} M$

| Sample ID | Conc. (nM) | KD (M) | ka (1/Ms) | ka Error | kd (1/s) | kd Error | Rmax | Rmax Error | R equilibrium |
|---|---|---|---|---|---|---|---|---|---|
| Antibody A | 43.52 | 1.84E-07 | 3.70E+05 | 1.34E+04 | 6.81E-02 | 6.28E-04 | 2.432 | 0.08423 | 0.4654 |
| Antibody A | 87.06 | 1.84E-07 | 3.70E+05 | 1.34E+04 | 6.81E-02 | 6.28E-04 | 2.262 | 0.06648 | 0.7268 |
| Antibody A | 174 | 1.84E-07 | 3.70E+05 | 1.34E+04 | 6.81E-02 | 6.28E-04 | 1.899 | 0.04305 | 0.9233 |

# FIG. 2-1

(C) MabSelect SuRe 1.9 Ligand-immobilized Chip
+ Tocilizumab-containing Solution

KD = $8.11 \times 10^{-9}$M

| Sample ID | Conc. (nM) | KD (M) | ka (1/Ms) | ka Error | kd (1/s) | kd Error | Rmax | Rmax Error | R equilibrium |
|---|---|---|---|---|---|---|---|---|---|
| Tocilizumab | 41.34 | 8.11E-09 | 3.46E+05 | 1.44E+03 | 2.81E-03 | 1.67E-05 | 2.084 | 0.01365 | 1.743 |
| Tocilizumab | 82.68 | 8.11E-09 | 3.46E+05 | 1.44E+03 | 2.81E-03 | 1.67E-05 | 1.799 | 0.005814 | 1.639 |
| Tocilizumab | 165.3 | 8.11E-09 | 3.46E+05 | 1.44E+03 | 2.81E-03 | 1.67E-05 | 1.722 | 0.00256 | 1.642 |

(D) MabSelect SuRe 1.9 Ligand-immobilized Chip
+ Antibody A-containing Solution

KD = n.d.

| Sample ID | Conc. (nM) | KD (M) | ka (1/Ms) | ka Error | kd (1/s) | kd Error | Rmax | Rmax Error | R equilibrium |
|---|---|---|---|---|---|---|---|---|---|
| Antibody A | 43.52 | -- | -- | -- | -- | -- | -- | -- | -- |
| Antibody A | 87.06 | -- | -- | -- | -- | -- | -- | -- | -- |
| Antibody A | 174 | -- | -- | -- | -- | -- | -- | -- | -- |

FIG. 2-2

# EP 3 954 696 A1

<table>
<tr><td colspan="2" align="center"><strong>INTERNATIONAL SEARCH REPORT</strong></td><td colspan="2">International application No.<br>PCT/JP2020/015904</td></tr>
</table>

**A. CLASSIFICATION OF SUBJECT MATTER**
Int. Cl. C07K1/22(2006.01)i, C07K16/00(2006.01)i
FI: C07K16/00 ZNA, C07K1/22

According to International Patent Classification (IPC) or to both national classification and IPC

**B. FIELDS SEARCHED**

Minimum documentation searched (classification system followed by classification symbols)
Int. Cl. C07K1/22, C07K16/00

Documentation searched other than minimum documentation to the extent that such documents are included in the fields searched
Published examined utility model applications of Japan       1922-1996
Published unexamined utility model applications of Japan     1971-2020
Registered utility model specifications of Japan             1996-2020
Published registered utility model applications of Japan     1994-2020

Electronic data base consulted during the international search (name of data base and, where practicable, search terms used)
JSTPlus/JMEDPlus/JST7580 (JDreamIII),
CAplus/REGISTRY/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2017/104783 A1 (CHUGAI SEIYAKU KABUSHIKI KAISHA) 22 June 2017, paragraphs [0345], [0347], [0507], [0601], [0645] | 1-11 |
| Y | JP 2007-252368 A (PROTENOVA CO., LTD.) 04 October 2007, claims 1-4, example 7 | 1-11 |
| Y | WO 2015/034000 A1 (PROTENOVA CO., LTD.) 12 March 2015, claims 1-10, paragraphs [0023]-[0034], example 3 | 1-11 |
| A | JP 2010-81866 A (NATIONAL INSTITUTE OF ADVANCED INDUSTRIAL SCIENCE AND TECHNOLOGY) 15 April 2010, abstract | 1-11 |

☒ Further documents are listed in the continuation of Box C.  ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>09.06.2020 | Date of mailing of the international search report<br>23.06.2020 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

| International application No. |
| --- |
| PCT/JP2020/015904 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | ZWOLAK, Adam et al., Rapid Purification of Human Bispecific Antibodies via Selective Modulation of Protein A Binding, Scientific Reports, 14 November 2017, vol. 7, no. 15521, pp. 1-11, abstract | 1-11 |

Form PCT/ISA/210 (continuation of second sheet) (January 2015)

**INTERNATIONAL SEARCH REPORT**
Information on patent family members

International application No.

PCT/JP2020/015904

| Patent Documents referred to in the Report | Publication Date | Patent Family | Publication Date |
|---|---|---|---|
| WO 2017/104783 A1 | 22.06.2017 | JP 2017-112997 A<br>JP 2017-148069 A<br>KR 10-2017-0085028 A<br>CA 3002422 A<br>AU 2016372934 A<br>KR 10-2018-0085711 A<br>CN 108473562 A<br>MX 2018007145 A<br>BR 112018011073 A<br>EA 201891420 A<br>SG 11201610812W A<br>TW 201726718 A<br>AR 107078 A<br>TW 201808992 A<br>SG 102017072678 A | |
| JP 2007-252368 A | 04.10.2007 | JP 2008-214350 A<br>US 2010/0286373 A1<br>claims 1-4, example 7<br>WO 2007/097361 A1<br>EP 1992692 A1 | |
| WO 2015/034000 A1 | 12.03.2015 | US 2016/0215027 A1<br>claims 1-10,<br>paragraphs [0059]-<br>[0073], example 3<br>EP 3042912 A1 | |
| JP 2010-81866 A | 15.04.2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H05504579 A
- WO 07114319 A
- WO 2017104783 A
- WO 200711431 A
- JP 2007252368 A
- WO 2015034000 A
- WO 2006019447 A
- WO 2006053301 A
- WO 2009086320 A
- WO 2009041613 A
- WO 2011122011 A
- WO 2012132067 A
- WO 2013046704 A
- WO 2013180201 A
- WO 2013180200 A
- WO 2009125825 A
- WO 2012073992 A
- WO 2013047752 A
- EP 1752471 A
- EP 1772465 A
- WO 2012016227 A

- WO 2014145159 A
- JP 2015021371 A
- JP 2015185254 A
- EP 239400 A
- WO 9602576 A
- WO 0941613 A
- WO 9312227 A
- WO 9203918 A
- WO 9402602 A
- WO 9425585 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A
- WO 9219759 A

**Non-patent literature cited in the description**

- **HINTON et al.** *J. Immunol.,* 2006, vol. 176 (1), 346-356
- **DALL'ACQUA et al.** *J. Biol. Chem.,* 2006, vol. 281 (33), 23514-23524
- **PETKOVA et al.** *Intl. Immunol.,* 2006, vol. 18 (12), 1759-1769
- **ZALEVSKY et al.** *Nat. Biotechnol.,* 2010, vol. 28 (2), 157-159
- **MILSTEIN ; KOHLER, G. ; MILSTEIN, C. et al.** *Methods Enzymol.,* 1981, vol. 73, 3-46
- **CARL, A.K. BORREBAECK ; JAMES, W. LARRICK.** THERAPEUTIC MONOCLONAL ANTIBODIES. MACMILLAN PUBLISHERS LTD, 1990
- **SATO, K. et al.** *Cancer Res.,* 1993, vol. 53, 851-856
- **TSURUSHITA N ; HINTON PR ; KUMAR S.** Design of humanized antibodies: from anti-Tac to Zenapax. *Methods,* May 2005, vol. 36 (1), 69-83
- **RAJPAL A. ; BEYAZ N ; HABER L ; CAPPUCCILLI G ; YEE H ; BHATT RR ; TAKEUCHI T ; LERNER RA ; CREA R.** A general method for greatly improving the affinity of antibodies by using combinatorial libraries. *Proc Natl Acad Sci USA.,* 14 June 2005, vol. 102 (24), 8466-71

- **EWERT S ; HONEGGER A ; PLUCKTHUN A.** Stability improvement of antibodies for extracellular and intracellular applications: CDR grafting to stable frameworks and structure-based framework engineering. *Methods,* October 2004, vol. 34 (2), 184-99
- **KIM SJ ; PARK Y ; HONG HJ.** Antibody engineering for the development of therapeutic antibodies. *Mol Cells,* 31 August 2005, vol. 20 (1), 17-29
- **HINTON PR ; XIONG JM ; JOHLFS MG ; TANG MT ; KELLER S ; TSURUSHITA N.** An engineered human IgG1 antibody with longer serum half-life. *J. Immunol.,* 01 January 2006, vol. 176 (1), 346-56
- **GHETIE V ; POPOV S ; BORVAK J ; RADU C ; MATESOI D ; MEDESAN C ; OBER RJ ; WARD ES.** Increasing the serum persistence of an IgG fragment by random mutagenesis. *Nat Biotechnol.,* July 1997, vol. 15 (7), 637-40
- *Farmaco,* 30 August 1999, vol. 54 (8), 497-516
- *Cancer J,* May 2008, vol. 14 (3), 154-69
- **NILSSON B.** *Protein Engineering,* vol. 1 (2), 107-113

• **JANSSON, J.C. ; RYDEN, L.** *Protein purification,* IS-BN 0-471-18626-0, 375-442